# EUROPÄISCHE PATENTSCHRIFT

(11) **EP 2 178 899 B1**
(45) Veröffentlichungstag und Bekanntmachung des Hinweises auf die Patenterteilung: **24.10.2012**
(21) Anmeldenummer: 08758989.1
(22) Anmeldetag: 04.06.2008
(51) Int. Cl.: C07J 41/00, C07J 53/00, A61P 5/34, A61K 31/56

(54) **17BETA-CYANO-18A-HOMO-19-NOR-ANDROST-4-EN-DERIVAT, DESSEN VERWENDUNG UND DAS DERIVAT ENTHALTENDE ARZNEIMITTEL**
17BETA-CYANO-18A-HOMO-19-NOR-ANDROST-4-ENE DERIVATIVE, USE THEREOF AND MEDICAMENTS CONTAINING SAID DERIVATIVE
DÉRIVÉ DE 17BÊTA-CYANO-18A-HOMO-19-NOR-ANDROST-4-ÈNE, SON UTILISATION, ET MÉDICAMENT CONTENANT CE DÉRIVÉ

(30) Priorität: 12.06.2007 DE 102007027636
(43) Veröffentlichungstag der Anmeldung: 28.04.2010
(73) Patentinhaber: Bayer Intellectual Property GmbH, 40789 Monheim (DE)
(72) Erfinder: KUHNKE, Joachim, 14482 Potsdam (DE); HUEBNER, Jan, 10317 Berlin (DE); BOHLMANN, Rolf, 14055 Berlin (DE); FRENZEL, Thomas, 65719 Hofheim (DE); KLAR, Ulrich, 13503 Berlin (DE); MENGES, Frederik, 69198 Schriesheim (DE); RING, Sven, 07749 Jena (DE); BORDEN, Steffen, 13355 Berlin (DE); MUHN, Hans-Peter, 13465 Berlin (DE); PRELLE, Katja, 13465 Berlin (DE)
(74) Vertreter: Bayer Intellectual Property GmbH
(86) Internationale Anmeldenummer: PCT/EP2008/004428
(87) Internationale Veröffentlichungsnummer: WO 2008/151745

(56) Entgegenhaltungen:
- WO-A-2006/072467
- DE-A1- 2 109 853
- DE-A1- 2 226 552
- GB-A- 1 277 265
- US-A- 3 043 833
- US-A- 3 994 937

## Beschreibung

Die Erfindung betrifft bestimmte 17ß-Cyano-18a-homo-19-nor-androst-4-en-Derivate, deren Verwendung sowie die Derivate enthaltende Arzneimittel mit gestagener Wirkung, beispielsweise zur Behandlung von prä-, peri- und postmenopausalen sowie von prämenstrueller Beschwerden.

Aus der Literatur sind Verbindungen mit gestagener, antimineralcorticoider, antiandrogener oder antiestrogener Wirkung auf Basis eines Steroidgerüstes bekannt, welche beispielsweise von 19-Nor-androst-4-en-3-on oder einem Derivat davon abgeleitet sind (die Nummerierung des Steroidgerüstes ist beispielsweise Fresenius/Görlitzer 3.Aufl. 1991 "Organisch-chemische Nomenklatur" S. 60 ff. zu entnehmen).

So offenbart WO 2006072467 A1 die als Gestagen wirkende Verbindung 6β,7β-15β,16β-Dimethylen-3-oxo-17-pregn-4-en-21,17β-carbolacton (Drospirenon), welche beispielsweise in einem oralen Kontrazeptivum sowie einem Präparat zur Behandlung postmenopausaler Beschwerden verwendet wurde. Aufgrund seiner vergleichsweise geringen Affinität zum Gestagenrezeptor und seiner vergleichsweise hohen Ovulationshemmdosis ist Drospirenon in dem Kontrazeptivum jedoch in der relativ hohen täglichen Dosis von 3 mg enthalten. Drospirenon zeichnet sich darüber hinaus auch dadurch aus, dass es zusätzlich zur gestagenen Wirkung über aldosteronantagonistische (antimineralcorticoide) sowie antiandrogene Wirkung verfügt. Diese beiden Eigenschaften machen Drospirenon in seinem pharmakologischen Profil dem natürlichen Gestagen Progesteron sehr ähnlich, welches aber anders als Drospirenon nicht ausreichend oral bioverfügbar ist. Um die zu verabreichende Dosis zu senken, werden in WO 2006072467 A1 weiter ein 18-Methyl-19-nor-17-pregn-4-en-21,17-carbolacton sowie diese enthaltende pharmazeutische Präparate vorgeschlagen, welche über eine höhere gestagene Potenz als Drospirenon verfügen.

Daneben offenbart beispielsweise US-A 3,705,179 Steroide, welche eine antiandrogene Aktivität aufweisen und sich zur Behandlung von Krankheiten eignen, die im Zusammenhang mit Androgenen stehen.

In DE 22 26 552 B2 sind weiter 17-Cyano-19-nor-androst-4-en-3-on-Verbindungen beschrieben, welche progestomimetische, antiandrogene sowie antiestrogene Wirkungen mit exogenem Charakter zeigen.

Die Aufgabe der vorliegenden Erfindung ist es, Verbindungen zur Verfügung zu stellen, die über eine starke Bindung an den Gestagenrezeptor verfügen. Außerdem sollen die Verbindungen bevorzugt auch eine antimineralcorticoide Wirkung aufweisen.

Diese Aufgabe wird durch die erfindungsgemäßen 17ß-Cyano-18a-homo-19-nor-androst-4-en-Derivate gemäß Anspruch 1, die Verwendung der erfindungsgemäßen Derivate gemäß Anspruch 11 sowie ein mindestens ein erfindungsgemäßes Derivat enthaltendes Arzneimittel gemäß Anspruch 13 gelöst. Vorteilhafte Ausführungsformen der Erfindung sind in den Unteransprüchen angegeben.

Die vorliegende Erfindung betrifft demnach ein 17ß-Cyano-18a-homo-19-nor-androst-4-en-Derivat mit der allgemeinen chemischen Formel 1 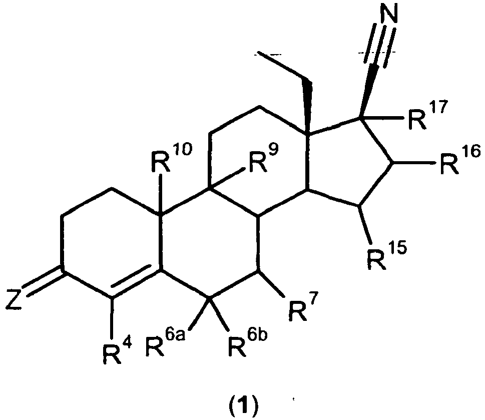 wobei
- Z: ausgewählt ist aus der Gruppe, umfassend O, zwei Wasserstoffatome, NOR und NNHSO₂R, worin R Wasserstoff oder C₁-C₄-Alkyl ist,
- R⁴: Wasserstoff oder Halogen ist,
ferner entweder:
- R^{6a}, R^{6b}: gemeinsam Methylen oder 1,2-Ethandiyl bilden oder R^{6a} Wasserstoff ist und R^{6b} aus der Gruppe ausgewählt ist, umfassend Wasserstoff, Methyl und Hydroxymethylen, und
- R⁷: ausgewählt ist aus der Gruppe, umfassend Wasserstoff, C₁-C₄-Alkyl, C₂-C₃-Alkenyl und Cyclopropyl,
oder:
R^{6a} Wasserstoff ist und R^{6b} und R⁷ entweder gemeinsam Methylen bilden oder unter Bildung einer Doppelbindung zwischen C⁶ und C⁷ entfallen,

- R⁹, R¹⁰: Wasserstoff sind oder unter Bildung einer Doppelbindung zwischen C⁹ und C¹⁰ entfallen,
- R¹⁵, R¹⁶: Wasserstoff sind oder gemeinsam Methylen bilden,
- R¹⁷: ausgewählt ist aus der Gruppe, umfassend Wasserstoff, C₁-C₄-Alkyl und Allyl,
wobei mindestens einer der Substituenten R⁴, R^{6a}, R^{6b}, R⁷, R¹⁵, R¹⁶ und R¹⁷ ungleich Wasserstoff ist oder R^{6b} und R⁷ unter Bildung einer Doppelbindung zwischen C⁶ und C⁷ entfallen
sowie deren Solvate, Hydrate und Salze.

Die Nummerierung des C-Gerüstes des erfindungsgemäßen Derivates mit der allgemeinen chemischen Formel **1** folgt in üblicher Weise der Nummerierung eines Steroid-Gerüstes, beispielsweise beschrieben in Fresenius, *a.a.O*. Die Nummerierung der in den Ansprüchen angegebenen Reste entspricht in analoger Weise ihrer Bindungsposition am C-Gerüst des Derivates. So bindet beispielsweise der Rest R⁴ an die C⁴-Position des erfindungsgemäßen Derivates.

Hinsichtlich der zu Z definierten Gruppen binden die Gruppen NOR und NNHSO₂R jeweils mit einer Doppelbindung über N an das C-Gerüst des Derivates gemäß =NOR bzw. =N-NH-SO₂R. OR in NOR und NHSO₂R in NNHSO₂R können syn- oder antiständig stehen.

Unter C₁-C₄-Alkyl ist jeweils ein geradkettiger oder verzweigter Alkylrest, nämlich Methyl, Ethyl, n-Propyl, iso-Propyl, n-Butyl, iso-Butyl oder tert.-Butyl, zu verstehen. Besonders bevorzugt sind Methyl, Ethyl und n-Propyl, vor allem die unverzweigten Reste. Besonders bevorzugt sind Methyl, Ethyl und n-Propyl. In 17α-Stellung gebundene Alkylreste können außerdem perfluoriert sein, so dass R¹⁷ in diesem Falle außerdem Trifluormethyl, Pentafluorethyl, n-Heptafluorpropyl, iso-Heptafluorpropyl, n-Nonafluorbutyl, iso-Nonafluorbutyl und tert-Nonafluorbutyl sein kann.

Unter C₂-C₃-Alkenyl ist vorzugsweise Vinyl oder Allyl zu verstehen.

Unter Halogen ist jeweils Fluor, Chlor, Brom oder Jod zu verstehen.

Die erfindungsgemäßen Derivate können auch in Form von Solvaten, insbesondere von Hydraten vorliegen, wobei die erfindungsgemäßen Verbindungen demgemäß polare Lösungsmittel, insbesondere von Wasser, als Strukturelement des Kristallgitters der erfindungsgemäßen Verbindungen enthalten. Das polare Lösungsmittel, insbesondere Wasser, kann in einem stöchiometrischen oder auch unstöchiometrischen Verhältnis vorliegen. Bei stöchiometrischen Solvaten, Hydraten spricht man auch von Hemi-, (Semi-), Mono-, Sesqui-, Di-, Tri-, Tetra-, Penta-, usw. Solvaten oder Hydraten.

Es wurde gefunden, dass die erfindungsgemäßen Verbindungen bzw. Derivate eine gute gestagene Wirkung in vivo aufweisen. Außerdem wirken einige interessante erfindungsgemäße Verbindungen als Antagonisten zum Mineralcorticoidrezeptor.

Bevorzugt sind erfindungsgemäße Derivate mit der vorgenannten allgemeinen chemischen Formel 1, in denen Z ausgewählt ist aus der Gruppe, umfassend O, NOH und NNHSO₂H. Besonders bevorzugt steht Z für O.

Unabhängig von der Auswahl von Z sind weiterhin erfindungsgemäße Derivate mit der vorgenannten allgemeinen chemischen Formel 1 bevorzugt, in denen folgende Varianten alternativ oder aber zumindest zum Teil gemeinsam vorkommen und unabhängig voneinander ausgewählt sind:
Vor allem bilden vorzugsweise R¹⁵ und R¹⁶ gemeinsam Methylen, wobei sowohl eine α-ständige als auch eine β-ständige Methylengruppe in diesen Positionen gebunden sein kann.

Ferner ist R⁴ vorzugsweise Wasserstoff oder Chlor.

Ferner bilden R^{6a} und R^{6b} vorzugsweise gemeinsam 1,2-Ethandiyl oder sind jeweils Wasserstoff.

Ferner ist R⁷ vorzugsweise ausgewählt ist aus der Gruppe, umfassend Wasserstoff und Methyl, wobei die Methylgruppe sowohl α-ständig als auch β-ständig sein kann.

Ferner bilden R^{6b} und R⁷ vorzugsweise gemeinsam Methylen, wobei die Methylengruppe sowohl α-ständig als auch β-ständig sein kann.

Ferner ist R¹⁷ vorzugsweise ausgewählt ist aus der Gruppe, umfassend Wasserstoff und Methyl.

Ferner können die Reste R^{6a}, R^{6b}, R⁷, R¹⁵ und R¹⁶ sowohl α- als auch β-ständig sein.

Besonders bevorzugt sind dabei die erfindungsgemäßen 17β-Cyano-18a-homo-19-nor-androst-4-en-Derivate, ausgewählt aus der Gruppe, umfassend

| | |
|---|---|
| 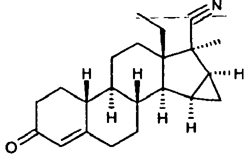 | 17β-Cyano-17α-methyl-15β,16β-methylen-18a-homo-19-nor-androst-4-en-3-on |
| 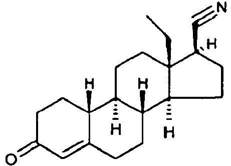 | 17β-Cyano-18a-homo-19-nor-androst-4-en-3-on |
| 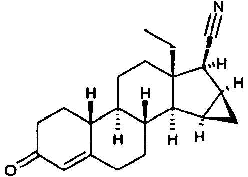 | 17β-Cyano-15β,16β-methylen-18a-homo-19-nor-androst-4-en-3-on |
| 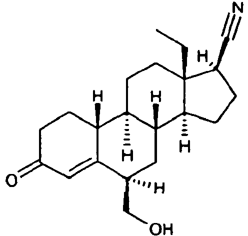 | 17β-Cyano-6β-hydroymethylen-18a-homo-19-nor-androst-4-en-3-on |
| 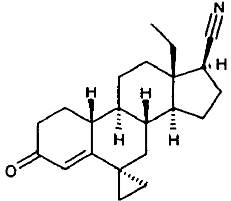 | 17β-Cyano-6,6-ethandiyl-18a-homo-19-nor-androst-4-en-3-on |
| 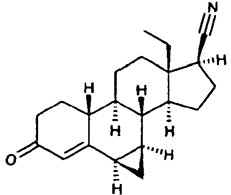 | 17β-Cyano-6β,7β-methylen-18a-homo-19-nor-androst-4-en-3-on |
| 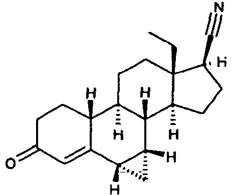 | 17β-Cyano-6α,7α-methylen-18a-homo-19-nor-androst-4-en-3-on |
| 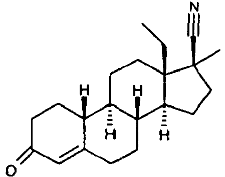 | 17β-Cyano-17α-methyl-18a-homo-19-nor-androst-4-en-3-on |
| 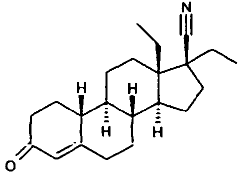 | 17β-Cyano-17α-ethyl-18a-homo-19-nor-androst-4-en-3-on |
| 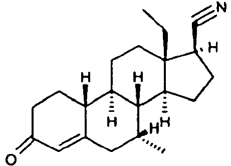 | 17β-Cyano-7α-methyl-18a-homo-19-nor-androst-4-en-3-on |
| 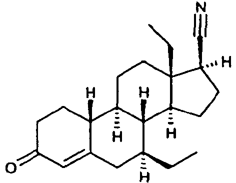 | 17β-Cyano-7β-ethyl-18a-homo-19-nor-androst-4-en-3-on |
| 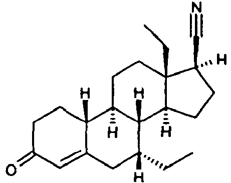 | 17β-Cyano-7α-ethyl-18a-homo-19-nor-androst-4-en-3-on |
| 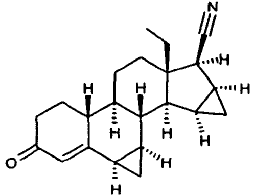 | 17β-Cyano-6β,7β; 15β,16β-bismethylen-18a-homo-19-nor-androst-4-en-3-on |
| 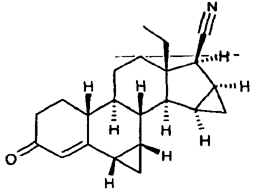 | 17β-Cyano-6α,7α; 15β,16β-bismethylen-18a-homo-19--nor-androst-4-en-3-on |
| 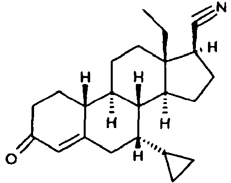 | 17β-Cyano-7α-cyclopropyl-18a-homo-19-nor-androst-4-en-3-on |
| 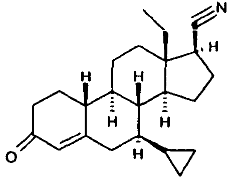 | 17β-Cyano-7β-cyclopropyl-18a-homo-19-nor-androst-4-en-3-on |
| 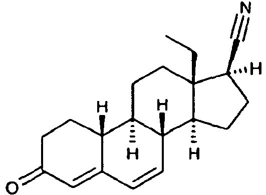 | 17β-Cyano-18a-homo-19-nor-androst-4,6-dien-3-on |
| 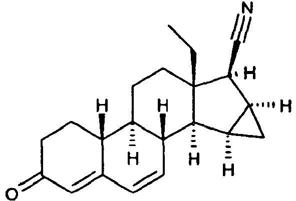 | 17β-Cyano-15β,16β-methylen-18a-homo-19-nor-androst-4,6-dien-3-on |
| 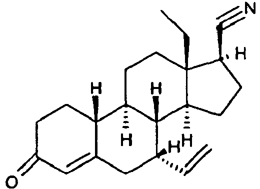 | 17β-Cyano-7α-vinyl-18a-homo-19-nor-androst-4-en-3-on |
| 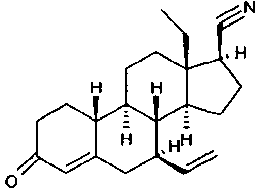 | 17β-Cyano-7β-vinyl-18a-homo-19-nor-androst-4-en-3-on |
| 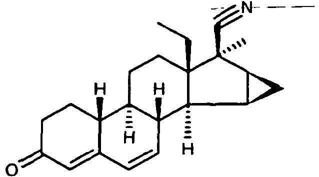 | 17β-Cyano-17α-methyl-15β,16β-methylen-18a-homo-19-nor-androsta-4,6-dien-3-on |
| 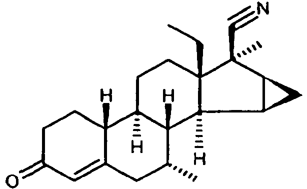 | 17βß-Cyano-7α,17α-bismethyl-15β,16β-methylen-18a-homo-19-nor-androst-4-en-3-on |
| 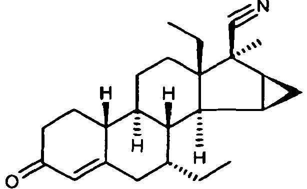 | 17β-Cyano-7α-ethyl-17α-methyl-15β,16β-methylen-18a-homo-19-nor-androst-4-en-3-on |
| 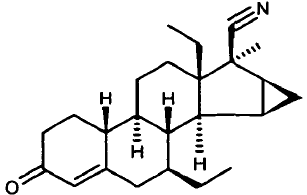 | 17β-Cyano-7β-ethyl-17α-methyl-15β,16β-methylen-18a-homo-19-nor-androst-4-en-3-on |
| 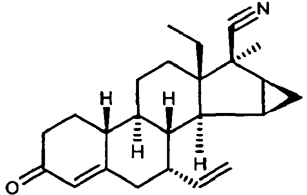 | 17β-Cyano-17α-methyl-15β,16β-methylen-7α-vinyl-18a-homo-19-nor-androst-4-en-3-on |
| 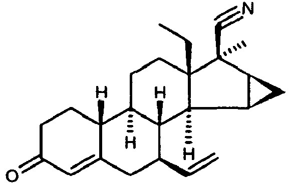 | 17β-Cyano-17α-methyl-15β,16β-methylen-7β-vinyl-18a-homo-19-nor-androst-4-en-3-on |
| 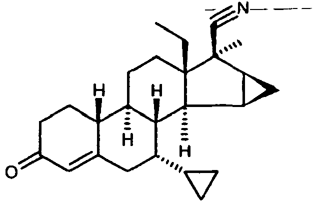 | 17β-Cyano-7α-cyclopropyl-17α-methyl-15β,16β-methylen-18a-homo-19-nor-androst-4-en-3-on |
| 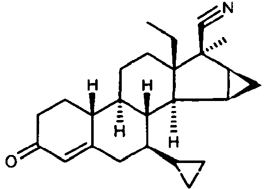 | 17β-Cyano-7β-cyclopropyl-17α-methyl-15β,16β-methylen-18a-homo-19-nor-androst-4-en-3-on |
| 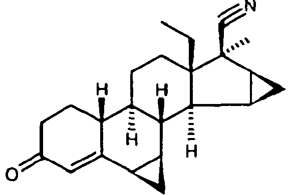 | 17β-Cyano-17α-methyl-6β,7β-15β,16β-bismethylen-18a-homo-19-nor-androst-4-en-3-on |
| 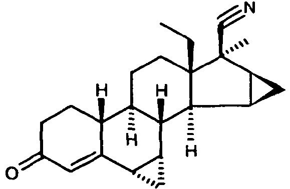 | 17β-Cyano-17α-methyl-6α,7α-15β,16β-bismethylen-18a-homo-19-nor-androst-4-en-3-on |
| 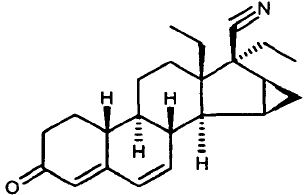 | 17β-Cyano-17α-ethyl-15β, 16β-methylen-18a-homo-19-nor-androsta-4,6-dien-3-on |
| 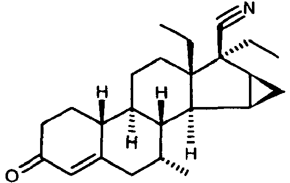 | 17β-Cyano-17α-ethyl-7α-methyl-15β,16β-methylen-18a-homo-19-nor-androst-4-en-3-on |
| 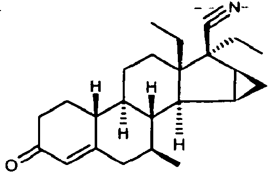 | 17β-Cyano-17α-ethyl-7β-methyl-15β,16β-methylen-18a-homo-19-nor-androst-4-en-3-on |
| 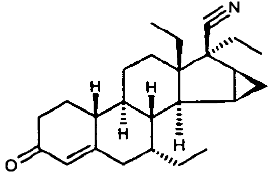 | 17β-Cyano-7α-,17α-bisethyl-15β,16β-methylen-18a-homo-19-nor-androst-4-en-3-on |
| 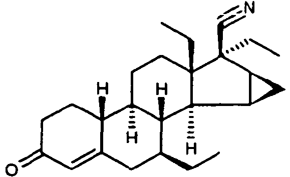 | 17β-Cyano-7ß,17α-bisethyl-15β,16β-methylen-18a-homo-19-nor-androst-4-en-3-on |
| 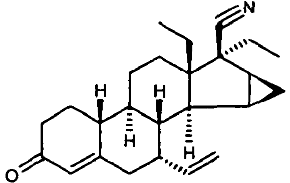 | 17β-Cyano-17α-ethyl-15β,16β-methylen-7α-vinyl-18a-homo-19-nor-androst-4-en-3-on |
| 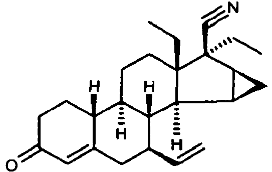 | 17β-Cyano-17α-ethyl-15β,16β-methylen-7β-vinyl-18a-homo-19-nor-androst-4-en-3-on |
| 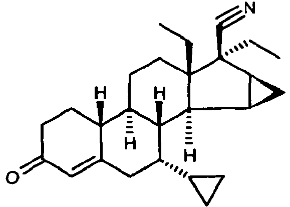 | 17β-Cyano-7α-cyclopropyl-17α-ethyl-15β,16β-methylen-18a-homo-19-nor-androst-4-en-3-on |
| 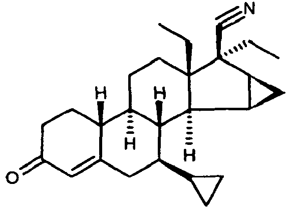 | 17β-Cyano-7β-cyclopropyl-17α-ethyl-15β,16β-methylen-18a-homo-19-nor-androst-4-en-3-on |
| 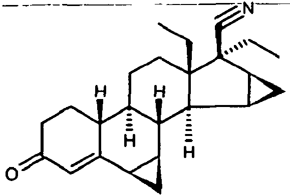 | 17β-Cyano-17α-ethyl-6β,7β-15β,16β-bismethylen-18a-homo-19-nor-androst-4-en-3-on |
| 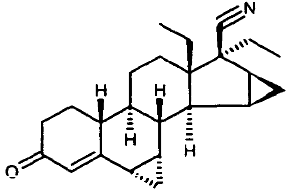 | 17β-Cyano-17α-ethyl-6α,7α-15β,16β-bismethylen-18a-homo-19-nor-androst-4-en-3-on |

Ganz besonders bevorzugt sind die 15α,16α- und die 15ß,16ß-Methylen-Derivate in der vorstehenden Liste.

Aufgrund ihrer gestagenen Wirksamkeit können die neuen Verbindungen mit der allgemeinen chemischen Formel 1 allein oder in Kombination mit Estrogenen in Arzneimitteln zur Kontrazeption verwendet werden.

Die erfindungsgemäßen Derivate eignen sich daher insbesondere zur Herstellung eines Arzneimittels zur oralen Kontrazeption und zur Behandlung von prä-, peri- und postmenopausalen Beschwerden, einschließlich der Verwendung in Präparaten für die Hormon-Substitutionstherapie (HRT).

Wegen ihres günstigen Wirkungsprofils sind die erfindungsgemäßen Derivate außerdem besonders gut geeignet zur Behandlung prämenstrueller Beschwerden, wie Kopfschmerzen, depressiver Verstimmungen, Wasserretention und Mastodynie.

Besonders bevorzugt ist die Verwendung der erfindungsgemäßen Derivate zur Herstellung eines Arzneimittels mit gestagener und antimineralcorticoider Wirkung.

Eine Behandlung mit den erfindungsgemäßen Derivaten findet bevorzugt am Menschen statt, kann aber auch an verwandten Säugetierspezies, wie beispielsweise an Hund und Katze, durchgeführt werden.

Zur Verwendung der erfindungsgemäßen Derivate als Arzneimittel werden diese mit mindestens einem geeigneten pharmazeutisch unbedenklichen Zusatzstoff, beispielsweise Trägerstoff, kombiniert. Der Zusatzstoff ist beispielsweise für die parenterale, vorzugsweise orale, Applikation geeignet. Es handelt sich dabei um pharmazeutisch geeignete organische oder anorganische inerte Zusatzmaterialien, wie zum Beispiel, Wasser, Gelatine, Gummi arabicum, Milchzucker, Stärke, Magnesiumstearat, Talk, pflanzliche Öle, Polyalkylenglykole usw. Die Arzneimittel können in fester Form, zum Beispiel als Tabletten, Dragees, Suppositorien, Kapseln, oder in flüssiger Form, zum Beispiel als Lösungen, Suspensionen oder Emulsionen, vorliegen. Gegebenenfalls enthalten sie darüber hinaus Hilfsstoffe, wie Konservierungs-, Stabilisierungs-, Netzmittel oder Emulgatoren, Salze zur Veränderung des osmotischen Druckes oder Puffer. Für die parenterale Applikation sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylbenzoat oder Benzylalkohol, zugesetzt werden. Es ist auch möglich, die erfindungsgemäßen Derivate in ein transdermales System einzuarbeiten und sie damit transdermal zu applizieren. Für die orale Applikation kommen insbesondere Tabletten, Dragees, Kapseln, Pillen, Suspensionen oder Lösungen in Frage.

Die Dosierung der erfindungsgemäßen Derivate in Kontrazeptionspräparaten soll 0,01 bis 10 mg pro Tag betragen. Die Tagesdosis bei der Behandlung prämenstrueller Beschwerden liegt bei etwa 0,1 bis 20 mg. Die erfindungsgemäßen gestagenen Derivate werden in Kontrazeptionspräparaten sowie in den Arzneimitteln zur Behandlung prämenstrueller Beschwerden vorzugsweise oral appliziert. Die tägliche Dosis wird vorzugsweise einmalig verabreicht.

Die gestagenen und estrogenen Wirkstoffkomponenten werden in Kontrazeptionspräparaten vorzugsweise zusammen oral appliziert. Die tägliche Dosis wird vorzugsweise einmalig verabreicht.

Als Estrogene kömmen synthetische Estrogene, vorzugsweise Ethinylestradiol, aber auch Mestranol in Betracht.

Das Estrogen wird in einer täglichen Menge verabfolgt, die der von 0,01 bis 0,04 mg Ethinylestradiol entspricht.

Als Estrogene in den Arzneimitteln zur Behandlung von prä-, peri- und postmenopausalen Beschwerden sowie für die Hormon-Substitutionstherapie kommen in erster Linie natürliche Estrogene zur Anwendung, vor allem das Estradiol oder dessen Ester, beispielsweise Estradiolvalerat, oder auch konjugierte Estrogene (CEEs = Conjugated Equine Estrogens).

Soweit die Herstellung der Ausgangsverbindungen hier nicht beschrieben ist, sind diese dem Fachmann bekannt oder analog zu bekannten Verbindungen oder hier beschriebenen Verfahren herstellbar. Die Isomerengernische können nach üblichen Methoden, wie beispielsweise Kristallisation, Chromatographie oder Salzbildung, in die Enantiomeren, E/Z-Isomeren bzw. Epimeren aufgetrennt werden.

Die erfindungsgemäßen Derivate mit der allgemeinen chemischen Formel 1 werden wie nachstehend beschrieben hergestellt.

Als Startmaterialien für die hier beschriebenen 17β-Cyano-18a-Homo-19-nor-androst-4-en-3-on-Derivate eignen sich diverse steroidale Ausgangsmaterialien wie beispielsweise 18a-Homo-19-Nor-androst-4-en-3,17-dion oder auch die teilreduzierten Analoga.

15β,16β-Methylen-3-methoxy-18a-homo-19-nor-androst-3,5-dien-17-on (WO 2006/072467 A1) eignet sich als Ausgangsmaterial für 15β,16β-methylenierte 17-Cyano-Derivate. 15α,16α-methylenierte Vorstufen, die geeignet für die Synthese der entsprechenden 17-Cyanosteroide sind, sind ebenfalls bekannt, z.B. 17β-hydroxy-15α,16α-methylen-18a-homo-19-nor-androst-4-en-3-on in DE-A 22 07 421 (1973).

Dem Fachmann selbstverständlich ist, dass bei den Beschreibungen der synthetischen Transformationen immer vorausgesetzt wird, dass gegebenenfalls am Steroidgerüst vorhandene sonstige funktionelle Gruppen in geeigneter Form geschützt sind.

Die Einführung eines Nitrils in Position 17 (C¹⁷) des Steroidgerüstes kann auf vielfältige Weise erfolgen. Hier kommen sowohl einstufige Verfahren als auch mehrstufige Varianten in Betracht. Bevorzugt sind hier Methoden, die letztlich den Austausch einer Sauerstofffunktion gegen Cyanid bedeuten. Viele in Betracht kommende Verfahrensvarianten sind beschrieben in Science of Synthesis Houben-Weyl Methods of Molecular Transformations Category 3 Volume 19 S. 197-213 (2004 Georg Thieme Verlag Stuttgart, New York) sowie in Houben-Weyl Methoden der organischen Chemie Band E5 Teil 2 S. 1318-1527 (1985 Georg Thieme Verlag Stuttgart, New York).

Als Einstufenverfahren bietet sich beispielsweise der direkte reduktive Austausch eines Carbonylsauerstoffes gegen eine Cyanogruppe an. Hierzu wird ein 17-Ketosteroid mit Tosylmethylisocyanid in geeigneten Lösemitteln wie etwa Dimethoxyethan, Dimethylsulfoxid, Ethern, Alkoholen oder auch deren Gemischen unter Verwendung geeigneter Basen, wie etwa Alkaliakoholaten, Alkalihydriden, Kallumhexamethyldisilazid, oder auch Alkaliamiden, wie etwa Lithiumdiisopropylamid, in einem Temperaturbereich von 0°C bis 100°C umgesetzt. Gegebenenfalls entstehende 17-Epimerengemische lassen sich durch Chromatographie, fraktionierte Kristallisation oder mit einer Kombination dieser Methoden trennen.

Auch der SN₂-artige Austausch einer geeigneten Abgangsgruppe an Position 17, wie etwa eines Halogenides (bevorzugt Jod oder Brom) oder auch eines Sulfonsäureesters eines 17-Alkoholes gegen Cyanid kommt in Betracht. Als Cyanidquellen werden bevorzugt anorganische Cyanide, wie Lithium-, Natrium- und Kaliumcyanid verwendet.

Als Beispiele für mehrstufige Varianten der Nitrileinführung seien die Folgenden genannt: ein 17-Keton wird mittels einer Wittig-Olefinierung in die entsprechende 17-Exomethylenverbindung überführt, welche nach Hydroborierung und Oxidation zum Aldehyd zum entsprechenden 17-Carbaldehydoxim umgesetzt werden kann. Dehydratisierung des Oxims führt dann zum 17-Nitril.

Die Einführung des Nitriles kann sowohl am Anfang einer Synthesesequenz als auch zu einem beliebigen späteren Zeitpunkt durchgeführt werden, vorausgesetzt, dass gegebenenfalls vorhandene weitere funktionelle Gruppen in geeigneter Weise geschützt sind.

Die 17-Cyanoverbindungen lassen sich gegebenenfalls alkylieren, was zu stereochemisch einheitlichen 17β-Cyano-17a-substituierten Derivaten führt. Hierzu wird in einem geeigneten Lösemittel, wie etwa Ethern, beispielsweise Tetrahydrofuran, das 17-Cyanosteroid_deprotoniert. Hier können diverse Basen verwendet werden, beispielsweise ein Alkaliamid, wie Lithiumdiisopropylamid. Nach Zusatz eines Alkylierungsmittels, wie etwa eines Alkyl- oder Alkenylhalogenides, und Aufarbeitung werden dann die 17ß-Cyano-17α-substituierten Derivate erhalten.

Exemplarisch sei das weitere synthetische Vorgehen an Hand des folgenden Syntheseschemas erläutert, wobei als Edukt die bereits beschriebene Verbindung 3 aufgeführt wird (vergleiche WO 2006072467 A1):

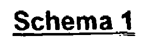

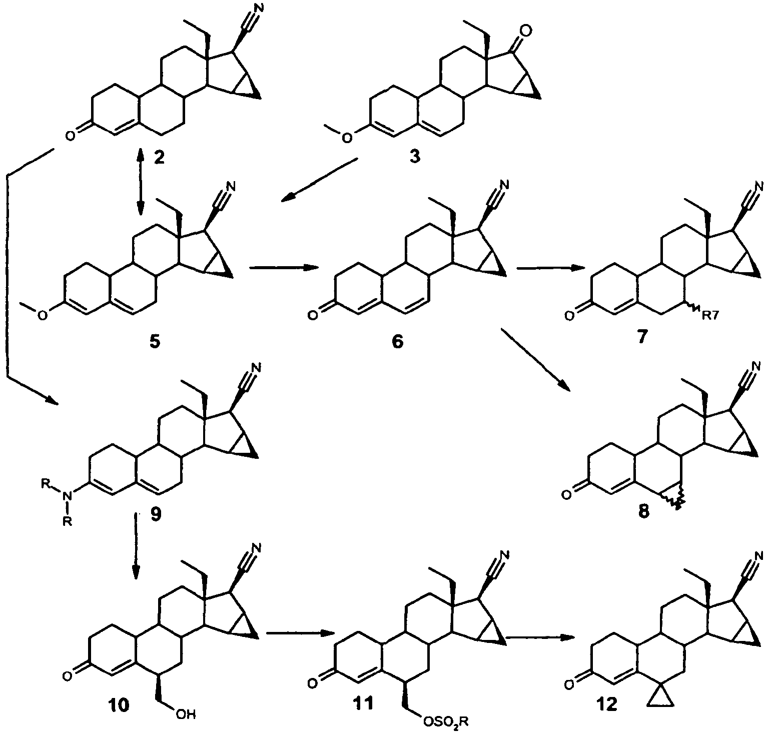

Nach einer der oben angegebenen Methoden lässt sich der Dienolether **3** in das 17-Cyano-Derivat **5** überführen. Die Einführung einer 6,7-Doppelbindung erfolgt über Bromierung des 3,5-Dienolethers **5** sowie anschließende Bromwasserstoffabspaltung (siehe z.B. J. Fried, J.A. Edwards, Organic Reactions in Steroid Chemistry, von Nostrand Reinhold Company 1972, S. 265-374).

Die Einführung eines Substituenten R⁴ kann zum Beispiel, ausgehend von einer Verbindung der Formel **2**, die durch säurekatalysierte Enoletherspaltung aus **5** erhältlich ist, durch Epoxidierung der 4,5-Doppelbindung mit Wasserstoffperoxid unter alkalischen Bedingungen und Umsetzung der entstandenen Epoxide in einem geeigneten Lösungsmittel mit Säuren mit der allgemeinen chemischen Formel H-R⁴ erreicht werden, wobei R⁴ ein Halogenatom oder ein Pseudohalogen sein kann, oder mit katalytischen Mengen Mineralsäure umsetzt und gegebenenfalls die erhaltenen 4-Bromverbindungen mit der allgemeinen chemischen Formel I (wobei R⁴ = Brom) mit 2,2-Difluor-2-(fluorsulfonyl)essigsäuremethylester in Dimethylformamid in Gegenwart von Kupfer(I)iodid umsetzt.

Die Dienoletherbromierung von Verbindung **5** kann z.B. analog zu der Vorschrift aus Steroids 1, 233 (1963) erfolgen. Die Bromwasserstoffabspaltung gelingt durch Erhitzen der 6-Bromverbindung mit basischen Reagenzien, wie z.B. LiBr oder Li₂CO₃ in aprotischen Lösungsmitteln wie Dimethylformamid bei Temperaturen von 50°C bis 120°C oder aber indem die 6-Bromverbindungen in einem Lösungsmittel, wie Collidin oder Lutidin, erhitzt werden zu Verbindung **6**. Liegt nicht der Enolether vor, sondern ein ungesättigtes Keton wie **2**, lässt sich dieses leicht in einen Dienolether vom Typ **5** überführen.

Verbindung 7 wird durch Methenylierung der 6,7-Doppelbindung nach bekannten Verfahren z.B. mit Dimethylsulfoxoniummethylid (siehe z.B. DE-A 11 83 500, DE-A 29 22 500, EP-A 0 019 690, US-A 4,291, 029; J. Am. Chem. Soc. 84, 867 (1962)) in eine Verbindung **8** umgewandelt, wobei ein Gemisch der α- und β-Isomeren erhalten wird, das z.B. durch Chromatographie in die einzelnen Isomeren getrennt werden kann.

Verbindungen vom Typ **7** können wie in den Beispielen beschrieben oder analog zu diesen Vorschriften unter Verwendung analoger zu den dort beschriebenen Reagenzien erhalten werden.

Die Synthese der spirocyclischen Verbindung **12** geht von **2** aus, welches zunächst in ein 3-Amino-3,5-dien-Derivat **9** überführt wird. Durch Umsetzung mit Formalin in alkoholischer Lösung wird das 6-Hydroxymethylen-Derivat **10** erhalten. Nach Überführung der Hydroxygruppe in eine Fluchtgruppe, wie etwa ein Mesylat, Tosylat (Verbindung **11**) oder auch Benzoat, lässt sich Verbindung **13** durch Umsetzung mit Trimethylsulfoxoniumiodid unter Verwendung von Basen, wie etwa Alkalihydroxiden, Alkalialkoholaten, in geeigneten Lösemitteln, wie etwa Dimethylsulfoxid, darstellen.

Zur Einführung einer 6-Methylengruppe kann Verbindung **10** mit z.B. Salzsäure in Dioxan/Wasser dehydratisiert werden. Auch aus **11** lässt sich 6-Methylen erzeugen (siehe DE-A 34 02 3291, EP-A 0 150 157, US-A 4,584,288; J. Med. Chem. 34, 2464 (1991)).

Eine weitere Möglichkeit zur Herstellung von 6- Methylenverbindungen besteht in der direkten Umsetzung der 4(5) ungesättigten 3-Ketone, wie Verbindung **2**, mit Acetalen des Formaldehyds in Gegenwart von Natriumacetat mit z.B. Phosphoroxychlorid oder Phosphorpentachlorid in geeigneten Lösungsmitteln, wie Chloroform (siehe z.B. K. Annen, H. Hofmeister, H. Laurent und R. Wiechert, Synthesis 34 (1982)).

Die 6-Methylenverbindungen können zur Darstellung von Verbindungen mit der allgemeinen chemischen Formel **1**, in denen R^{6a} gleich Methyl ist und R^{6b} und R⁷ unter Bildung einer Doppelbindung zwischen C⁶ und C⁷ entfallen, genutzt werden.

Hierzu kann man z.B. ein in Tetrahedron 21, 1619 (1965) beschriebenes Verfahren anwenden, bei dem eine Isomerisierung der Doppelbindung durch Erwärmen der 6-Methylenverbindungen in Ethanol mit 5% Palladium-Kohle-Katalysator, der entweder mit Wasserstoff oder durch Erwärmen mit einer geringen Menge Cyclohexen vorbehandelt wurde, erzielt wird. Die Isomerisierung kann auch mit einem nicht vorbehandelten Katalysator erfolgen, wenn zur Reaktionsmischung eine geringe Menge Cyclohexen zugesetzt wird. Das Auftreten geringer Anteile hydrierter Produkte kann durch Zugabe eines Überschusses an Natriumacetat verhindert werden.

Die Darstellung von 6-Methyl-4,6-dien-3-on-Derivaten kann aber auch direkt erfolgen (siehe K. Annen, H-Hofmeister, H. Laurent und R. Wiechert, Lieb. Ann. 712 (1983)).

Verbindungen, in denen R^{6b} eine α-Methylfunktion darstellt, können aus den 6-Methylenverbindungen durch Hydrierung unter geeigneten Bedingungen dargestellt werden. Die besten Ergebnisse (selektive Hydrierung der exo-Methylenfunktion) werden durch Transfer-Hydrierung erreicht (J. Chem. Soc. 3578 (1954)). Erhitzt man die 6-Methylenderivate in einem geeigneten Lösungsmittel, wie z.B. Ethanol, in Gegenwart eines Hydriddonators, wie z.B. Cyclohexen, so gelangt man in sehr guten Ausbeuten zu 6α-Methylderivaten. Geringe Anteile an 6β-Methylverbindung können sauer isomerisiert werden (Tetrahedron 1619 (1965)).

Auch die gezielte Darstellung von 6β-Methylverbindungen ist möglich. Hierfür werden die 4-En-3-one, wie etwa Verbindung **2**, z.B. mit Ethylenglycol, Trimethylorthoformiat in Dichlormethan in Gegenwart katalytischer Mengen einer Säure, z.B. p-Toluolsulfonsäure, zu den entsprechenden 3-Ketalen umgesetzt. Während dieser Ketalisierung isomerisiert die Doppelbindung in die Position 5 (C⁵). Eine selektive Epoxidierung dieser 5-Doppelbindung gelingt z.B. durch Verwendung organischer Persäuren, z.B. m-Chlorperbenzoesäure, in geeigneten Lösungsmitteln, wie Dichlormethan. Alternativ hierzu kann die Epoxidierung auch mit Wasserstoffperoxid in Gegenwart von z.B. Hexachloraceton oder 3-Nitrotrifluoracetophenon erfolgen. Die gebildeten 5,6α-Epoxide können dann unter Verwendung entsprechender Alkylmagnesiumhalogenide oder Alkyllithiumverbindungen axial geöffnet werden. Man gelangt so zu 5α-Hydroxy-6β-Alkylverbindungen. Die Spaltung der 3-Ketoschutzgruppe kann unter Erhalt der 5α-Hydroxyfunktion durch Behandeln unter milden sauren Bedingungen (Essigsäure oder 4 n Salzsäure bei 0°C) erfolgen. Basische Eliminierung der 5α-Hydroxyfunktion mit z.B. verdünnter wässriger Natronlauge ergibt die 3-Keto-4-en-Verbindungen mit einer β-ständigen 6-Alkylgruppe. Alternativ hierzu ergibt die Ketalspaltung unter drastischeren Bedingungen (wässrige Salzsäure oder eine andere starke Säure) die entsprechenden 6α-Alkylverbindungen.

Die erhaltenen Verbindungen mit der allgemeinen chemischen Formel I, in denen Z für ein Sauerstoffatom steht, können durch Umsetzung mit Hydroxylaminhydrochlorid in Gegenwart eines tertiären Amins bei Temperaturen zwischen -20 und +40°C in ihre entsprechenden Oxime überführt werden (allgemeine chemische Formel I mit Z in der Bedeutung von NOH, wobei die Hydroxygruppe syn- oder antiständig sein kann). Geeignete tertiäre Basen sind beispielsweise Trimethylamin, Triethylamin, Pyridin, N,N-Dimethylaminopyridin, 1,5- Diazabicyclo[4.3.0]non-5-en (DBN) und 1,5- Di-azabicyclo[5.4.0]undec-5-en (DBU), wobei Pyridin bevorzugt ist. Dies geht analog wie es in WO-A 98/24801 für die Herstellung entsprechender 3-Oxyimino-Derivate des Drospirenons beschrieben ist.

Die Entfernung der 3-Oxogruppe zur Herstellung eines Endprodukts mit der allgemeinen chemischen Formel **1** mit Z in der Bedeutung von zwei Wasserstoffatomen kann beispielsweise nach der in DE-A 28 05 490 angegebenen Vorschrift durch reduktive Spaltung eines Thioketals der 3-Ketoverbindung erfolgen.

Die nachfolgenden Beispiele dienen der näheren Erläuterung der Erfindung:
Die erfindungsgemäßen Verbindungen zeichnen sich überraschenderweise durch starke gestagene Wirksamkeit aus und sind im Schwangerschaftserhaltungs-Test an der Ratte nach subcutaner Applikation stark wirksam.

### Durchführung des Schwangerschaftserhaltungstests an der Ratte:

In graviden Ratten induziert die Entfernung der Corpora lutea oder Kastration einen Abort. Durch die exogene Zufuhr von Progestinen (Gestagenen) in Kombination mit einer geeigneten Dosis eines Estrogens gelingt die Aufrechterhaltung der Schwangerschaft. Der Schwangerschaftserhaltungstest an ovarektomierten Ratten dient zur Bestimmung der peripheren gestagenen Aktivität einer Verbindung.

Ratten wurden während des Proestrus über Nacht angepaart. Die Anpaarung wurde am Morgen des darauf folgenden Tages durch die Begutachtung eines Vaginalabstriches kontrolliert. Die Anwesenheit von Spermien wurde dabei als Tag 1 einer beginnenden Schwangerschaft bewertet. Am Tag 8 der Schwangerschaft wurden die Tiere unter Etheranesthesie ovarektomiert. Die Behandlung mit Testverbindung und exogenem Estrogen (Estron, 5 µg/kg/Tag) wurde von Tag 8 bis Tag 15 oder Tag 21 der Schwangerschaft einmal täglich subkutan ausgeführt. Die erste Anwendung am Tag 8 wurde zwei Stunden vor der Kastration ausgeführt. Intakte Kontrolltiere erhielten ausschließlich Vehikel.

### Auswertung:

Am Ende des Versuches (Tag 15 oder Tag 21) wurden die Tiere unter CO₂-Atmosphäre getötet, und es wurden lebende Föten (Föten mit schlagendem Herz) und Implantationsstellen (frühe Resorptionen und tote Föten einschließlich Autolyse und atrophische Plazenten) in beiden uterinen Hörnern gezählt. Am Tag 22 konnten Föten außerdem auf Missbildungen untersucht werden. In Uteri ohne Föten oder Implantationsstellen wurde die Anzahl von Nidationsstellen durch Anfärben mit 10%iger Ammoniumsulfid-Lösung ermittelt. Die Schwangerschaftserhaltungsrate wurde als Quotient aus der Anzahl der lebenden Föten und der gesamten Anzahl von Nidationsstellen (sowohl resorbierte und tote Föten als auch Nidationsstellen) berechnet. Für bestimmte Testsubstanzen wurden die in Tabelle 1 angegebenen die Schwangerschaft erhaltenden Dosen (ED50) ermittelt. Für Drospirenon beträgt dieser Wert 3,5 mg/kg/Tag.

Die erfindungsgemäßen Derivate mit der allgemeinen chemischen Formel 1 verfügen über eine sehr starke gestagene Wirksamkeit. Es wurde außerdem gefunden, dass die erfindungsgemäßen Derivate *in vitro* antimineralcorticoide Wirkung zeigen. Sie sollten deshalb *in vivo* kaliumretinierende, natriuretische (antimeralcorticoide) Wirkung besitzen. Diese Eigenschaften wurden mit dem nachfolgend beschriebenen Test ermittelt:
Zur Kultivierung der für den Assay verwendeten Zellen wurde als Kultivierungsmedium DMEM (Dulbecco's Modified Eagle Medium: 4500 mg/ml Glukose; PAA, #E15-009) mit 10% FCS (Biochrom, S0115, Charge #615B), 4 mM L-Glutamin, 1 % Penicillin/Streptomycin, 1 mg/ml G418 und 0,5 µg/ml Puromycin verwendet.

Reporter-Zelllinien wurden in einer Dichte von 4 x 104 Zellen pro Vertiefung in weißen, undurchsichtigen Gewebekulturplatten mit jeweils 96 Vertiefungen angezüchtet (PerkinElmer, #P12-106-017) und in 6 % DCC-FCS (Aktivkohle behandeltes Serum, zur Entfernung im Serum enthaltener störender Komponenten) gehalten. Die zu untersuchenden Verbindungen wurden acht Stunden später zugegeben, und die Zellen wurden mit den Verbindungen 16 Stunden lang inkubiert. Die Versuche wurden dreifach ausgeführt. Am Ende der Inkubation wurde das Effektor enthaltende Medium entfernt und durch Lysis-Puffer ersetzt. Nachdem Luciferase-Assay-Substrat (Promega, #E1501) zugegeben worden war, wurden die Platten mit den 96 Vertiefungen dann in ein Mikroplatten-Luminometer (Pherastar, BMG labtech) eingeführt, und die Lumineszenz wurde gemessen. Die IC50-Werte wurden unter Verwendung einer Software zur Berechnung von Dosis-Wirkungsbeziehungen ausgewertet. In Tabelle 1 sind Versuchsergebnisse wiedergegeben:

**Tabelle 1**

| Verbindung | MR Antagonismus IC50 [nM] | MR Antagonismus Wirksamkeit [% des Maximaleffektes] | PR *in vivo* ED50 [mg/kg/d s.c.] |
|---|---|---|---|
| 17β-Cyano-15β,16β-methylen-18a-homo-19-nor-androst-4-en-3-on | 18,0 | 103,44 | 0,01 |
| 17β-Cyano-7a-methyl-18a-homo-19-nor-androst-4-en-3-on | 16,0 | 99,07 | |
| 17β-Cyano-6β,7β; 15β,16β-bismethylen-18a-homo-19-nor-androst-4-en-3-on | 9,3 | 97,52 | 0,1 |
| 17β-Cyano-17α-methyl-6α,7α-15β,16β-bismethylen-18a-homo-19-nor-androst-4-en-3-on | 100 | 89,49 | 1,1 |
| 17β-Cyano-18a-homo-19-nor-androst-4-en-3-on | 9,1 | 94, 48 | 2,3 |
| 17β-Cyano-17α-methyl-15β,16β-methylen-18a-homo-19-nor-androst-4-en-3-on | 0,48 | 64,87 | 0,1 |

Die nachfolgenden Beispiele zur Synthese bevorzugter Erfindung dienen zur weiteren Veranschaulichung der Erfindung. Die in den einzelnen Synthesebeispielen offenbarten neuen Zwischenprodukte sind ebenso wie die erfindungsgemäßen 17β-Cyano-18a-homo-19-nor-androst-4-en-Derivate erfindungswesentlich.

HPLC-Trennungen wurden an chiraler Normalphase durchgeführt, wobei als stationäre Phase in der Regel Chiralpak AD-H 5µ Verwendung fand. Üblicherweise wurde mit einem Gemisch aus Hexan und Ethanol eluiert. In einigen Fällen fanden aber auch andere Eluentengemische, wie etwa Gemische aus Methanol und Ethanol Verwendung:

### Beispiel 1

### 17ß-Cyano-18a-homo-19-nor-androst-4-en-3-on

### 1a.

### 3-Methoxy-18a-homo-19-nor-androst-3(4),5(6)-dien-17-on

50 g 18a-Homo-19-nor-androst-4-en-3,17-dion wurden in 1 l Methanol und 175 ml Orthoameisensäuretrimethylester gelöst. Unter Rühren bei 25°C wurden 250 mg p-Toluolsulfonsäure zugegeben. Nach kurzer Zeit fiel das Produkt aus. Es wurde 1 h bei 25°C und 1 h bei -5°C gerührt. Es wurde mit Pyridin neutralisiert, abgesaugt und man erhielt 3-Methoxy-18a-homo-19-nor-androst-3(4),5(6)-dien-17-on.

### 1b.

### 3-Methoxy-18a-homo-19-nor-androst-3(4),5(6)-dien-17(S)-spiro-1',2'-oxiran

50 g 3-Methoxy-18a-homo-19-nor-androst-3(4),5(6)-dien-17-on wurden bei 25°C in 1 l Dimethylformamid aufgenommen. Dann wurden 68 g Trimethylsulfoniumiodid sowie 41 g Kalium-tert-butylat unter Rühren zugegeben, wobei die Temperatur bei ca. 20 - 25°C gehalten wurde. Nach 90 min wurde die Reaktionslösung in 2 110 %ige Ammoniumchloridlösung eingerührt und 30 min gerührt. Das ausgefallene Produkt wurde abgesaugt, mit Wasser gewaschen und trockengezogen. Man erhielt 3-Methoxy-18a-homo-19-nor-androst-3(4),5(6)-dien-17(S)-spiro-1',2'-oxiran.
MS: M+1 = 315,3

### 1c.

### 17ß-Hydroxy-17α-azidomethyl-3-methoxy-18a-homo-19-nor-androst-3(4),5(6)-dien

50 g 3-Methoxy-18a-homo-19-nor-androst-3(5),5(6)-dien-17(S)-spiro-1',2'-oxiran wurden in 1,5 l Ethylenglykol unter Rühren suspendiert, mit 90 g Natriumazid versetzt und 9 h bei 110 - 120°C gerührt. Nach dem Abkühlen wurde auf 3 l Wasser gegossen, abgesaugt und aus Methanol umkristallisiert. Man erhielt 17ß-Hydroxy-17α-azidomethyl-3-methoxy-18a-homo-19-nor-androst-3(4),5(6)-dien.
MS : M+1 = 358,3

### 1d.

### 3-Methoxy-18a-homo-19-nor-androst-3,5-dien-17β-carboxaldehyd

50 g 17ß-Hydroxy-17α-azidomethyl-3-methoxy-18a-homo-19-nor-androst-3,5-dien wurden in 450 ml Dichlormethan gelöst und unter Rühren langsam, bei 22°C, mit 68 g Triphenylphosphin versetzt. Es wurde 12 h gerührt und dann zur Trockene eingeengt. Man erhielt 3-Methoxy-18a-homo-19-nor-androst-3,5-dien-17ß-carboxaldehyd, welches ohne weitere Reinigung in die Folgestufe eingesetzt wurde.

### 1e.

### 18a-homo-20-Hydroxyimino-21,19-dinor-pregn-4-en-3-on

Das Rohprodukt aus der Vorstufe, 3-methoxy-18a-homo-19-nor-androst-3,5-dien-17ß-carboxaldehyd, wurde in 400 ml Pyridin gelöst und bei 22°C, unter Rühren, mit einer Lösung von 15 g Hydroxylaminhydrochlorid in 150 ml Pyridin versetzt. Danach wurde 2 h auf 60°C erwärmt, und die Lösung wurde auf 22°C abkühlen gelassen. Mit konzentrierter Salzsäure wurde ein pH Wert von 1-2 eingestellt, dann wurde mit 500 ml Wasser verdünnt, mit Essigester extrahiert und eingeengt. Der Rückstand wurde zur Reinigung an Kieselgel chromatographiert. Man erhielt 18a-homo-20-Hydroxyimino-21,19-dinor-pregn-4-en-3-on.

### 1f.

### 17ß-Cyano-18a-homo-19-nor-androst-4-en-3-on

4g 18a-homo-20-Hydroxyimino-21,19-dinor-pregn-4-en-3-on wurden in 40 ml Pyridin gelöst, und bei 10°C wurden 6,5 ml Methansulfonsäurechlorid zugetropft. Nach 1 h wurde auf 400 ml Wasser gegossen, mit Essigsäureethylester extrahiert und eingeengt. Der Rückstand wurde zur Reinigung an Kieselgel chromatographiert und aus Methyltertiärbutylether umkristallisiert. Man erhielt 17ß-Cyano-18a-homo-19-norandrost-4-en-3-on.
¹H-NMR (300 MHz, CDCl₃ TMS als interner Standard, ausgewählte Signale): δ = 1,12 (m, CH3-CH2), 5,83 (s, 4-H)
MS : M+1=298

### Beispiel 2

### 17ß-Cyano-18a-homo-19-nor-androst-4,6-dien-3-on

### 2a.

### 17ß-Cyano-3-methoxy-18a-homo-19-nor-androst-3,5(6)-dien

3,3g 17ß-Cyano-18a-homo-19-nor-androst-4-en-3-on wurden analog zu der in Beispiel 1a angegebenen Vorschrift umgesetzt. Man erhielt 17ß-Cyano-3-methoxy-18a-homo-19-nor-androst-3,5(6)-dien, welches roh weiter umgesetzt wurde.

### 2b.

### 17ß-Cyano-18a-homo-19-nor-androst-4,6-dien-3-on

Das Rohprodukt aus Beispiel 2a wurde in 100 ml 1-Methyl-2-pyrrolidon suspendiert. Es wurde nacheinander bei 0°C mit 4 ml einer 10%igen Natrumacetatlösung sowie bei dieser Temperatur mit 1,6 g 1,3-Dibrom-5,5-dimethylhydantoin portionsweise versetzt, 0,5 Stunden bei 0°C (Eisbad) gerührt, mit 1,5 g Lithiumbromid sowie 1,3 g Lithiumcarbonat versetzt, und 3,5 Stunden bei 100°C Badtemperatur gerührt. Anschließend wurde in Eiswasser / Kochsalz eingerührt, der Niederschlag wurde abfiltriert. Man erhielt 17ß-Cyano-18a-homo-19-nor-androst-4,6-dien-3-on.
¹H-NMR (300 MHz, CDCl₃ TMS als interner Standard, ausgewählte Signale): δ = 1,14(m,3H, CH3-CH2), 5,78(s,1H, H-4), 6,13(m,1H,H-6), 6,20(m,1H,H-7)
MS : M+1 = 296

### Beispiel 3

### 17ß-Cyano-7α-methyl-18a-homo-19-nor-androst-4-en-3-on

Zu einer Lösung von 1,0 g 17ß-Cyano-18a-homo-19-nor-androst-4,6-dien-3-on in 50 ml Tetrahydrofuran wurden bei Raumtemperatur 67 mg Kupfer-I-chlorid gegeben, und es wurde 10 Minuten gerührt, bevor auf -15°C abgekühlt wurde, dann wurde mit 450 mg Aluminiumchlorid versetzt, 30 Minuten bei dieser Temperatur gerührt, mit 4,5 ml Methylmagnesiumbromidlösung (3 M in Tetrahydrofuran) tropfenweise versetzt und eine Stunde bei -15°C gerührt. Zur Aufarbeitung wurde die Reaktionsmischung mit bei -15°C mit 30 ml 2 M Salzsäure versetzt, 0,5 Stunden bei Raumtemperatur gerührt, auf Wasser gegeben, dreimal mit Essigester extrahiert, über Natriumsulfat getrocknet, im Vakuum eingeengt, und an Kieselgel mit Hexan / Ethylacetat chromatographiert. Man erhielt 17ß-Cyano-7α-methyl-18a-homo-19-nor-androst-4-en-3-on.
¹H-NMR (300 MHz, CDCl₃ TMS als interner Standard, ausgewählte Signale): δ = 0,77 (d,3H,J=6,97, 7-CH3), 1,13(m,3H, CH3-CH2), 5,84(s,1H, H-4)

### Beispiel 4

### 17ß-Cyano-7α-ethyl-18a-homo-19-nor-androst-4-en-3-on und 17ß-Cyano-7ß-ethyl-18a-homo-19-nor-androst-4-en-3-on

Nach der Methode des Beispiels 3 mit Ethylmagnesiumbromid in Ether anstelle des Methylmagnesiumbromids erhielt man nach HPLC als Fraktion I 17ß-Cyano-7α-ethyl-18a-homo-19-nor-androst-4-en-3-on und als Fraktion II 17ß-Cyano-7ß-ethyl-18a-homo-19-nor-androst-4-en-3-on.

### 17ß-Cyano-7α-ethyl-18a-homo-19-nor-androst-4-en-3-on:

¹H-NMR (300 MHz, CDCl₃ TMS als interner Standard, ausgewählte Signale): δ = 0,87 (m.3H, 7-CH3-CH2), 1,13(m,3H, CH3-CH2), 5,85(s,1H, H-4)

### 17ß-Cyano-7ß-ethyl-18a-homo-19-nor-androst-4-en-3-on:

¹H-NMR (300 MHz, CDCl₃ TMS als interner Standard, ausgewählte Signale): δ = 0,87 (m.3H, 7-CH3-CH2), 1,12(m,3H, CH3-CH2), 5,82(s,1H, H-4)

### Beispiel 5

### 17ß-Cyano-7α-vinyl-18a-homo-19-nor-androst-4-en-3-on und 17ß-Cyano-7ß-vinyl-18a-homo-19-nor-androst-4-en-3-on

Nach der Methode des Beispiels 3 mit Vinylmagnesiumbromid anstelle des Methylmagnesiumbromids erhielt man nach HPLC als Fraktion I 17ß-Cyano-7α-vinyl-18a-homo-19-nor-androst-4-en-3-on und als Fraktion II 17ß-Cyano-7ß-vinyl-18a-homo-19-nor-androst-4-en-3-on.

### 17ß-Cyano-7α-vinyl-18a-homo-19-nor-androst-4-en-3-on:

¹H-NMR (300 MHz, CDCl₃ TMS als interner Standard, ausgewählte Signale): δ = 1,13(m,3H, CH3-CH2),5,08(m,2H, CH2=CH), 5,72 (m,1H,CH2=CH) 5,84(s,1H, H-4)

### 17ß-Cyano-7ß-vinyl-18a-homo-19-nor-androst-4-en-3-on:

¹H-NMR (300 MHz, CDCl₃ TMS als interner Standard, ausgewählte Signale): δ = 1,12(m,3H, CH3-CH2),4,98(m,2H, CH2=CH), 5,70 (m,1H,CH2=CH) 5,83(s,1H, H-4)

### Beispiel 6

### 17ß-Cyano-7α-cyclopropyl-18a-homo-19-nor-androst-4-en-3-on und 17ß-Cyano-7ß-cyclopropyl-18a-homo-19-nor-androst-4-en-3-on

Nach der Methode des Beispiels 3 mit Cyclopropylmagnesiumbromid anstelle des Methylmagnesiumbromids erhielt man nach der HPLC als Fraktion I 17ß-Cyano-7α-cyclopropyl-18a-homo-19-nor-androst-4-en-3-on und als Fraktion II 17ß-Cyano-7ß-cyclopropyl-18a-homo-19-nor-androst-4-en-3-on.

### 17ß-Cyano-7α-cyclopropyl-18a-homo-19-nor-androst-4-en-3-on:

¹H-NMR (300 MHz, CDCl₃ TMS als interner Standard, ausgewählte Signale): δ = - 0,05(m,1H,cyclopropyl), 0,26(m,1H,cyclopropyl), 0.47(m,3H, cyclopropyl), 1,13(m,3H, CH3-CH2), 5,88(s,1H, H-4)

### 17ß-Cyano-7ß-cyclopropyl-18a-homo-19-nor-androst-4-en-3-on:

¹H-NMR (300 MHz, CDCl₃ TMS als interner Standard, ausgewählte Signale): δ = 0,13(m,1H,cyclopropyl), 0,28(m,1H,cyclopropyl), 0,58(m,3H, cyclopropyl), 1,14(m,3H, CH3-CH2), 5,81(s,1H, H-4)

### Beispiel 7

### 17ß-Cyano-6ß-hydroxymethyl-18a-homo-19-nor-androst-4-en-3-on

3 g 17ß-Cyano-18a-homo-19-nor-androst-4-en-3-on wurden in 16 ml Methanol aufgenommen, mit 1,6 ml Pyrrolidin versetzt und 1 h am Rückfluss erwärmt. Nach dem Abkühlen wurde abgesaugt, mit wenig kaltem Methanol nachgewaschen und trockengezogen. Das Kristallisat wurde in 30 ml Benzol und 60 ml Ethanol gelöst, 3,1 ml 30 %ige Formaldehydlösung wurden zugegeben. Nach 2 h Rühren bei Raumtemperatur wurde zur Trockene eingeengt und an Kieselgel chromatographiert. Man erhielt 17ß-Cyano-6ß-hydroxymethyl-18a-homo-19-nor-androst-4-en-3-on.
¹H-NMR (300 MHz, CDCl₃ TMS als interner Standard, ausgewählte Signale): δ = 1,12(m,3H, CH3-CH2),3,67(m,2H,CH2OH) 5,90(s,1H, H-4)

### Beispiel 8

### 17ß-Cyano-6,6-ethylen-18a-homo-19-nor-androst-4-en-3-on

Zu einer Lösung von 1,74 g 17ß-Cyano-6ß-hydroxymethyl-18a-homo-19-nor-androst-4-en-3-on in 20 ml Pyridin wurden in einer Portion 2,93 g Tosylchlorid gegeben, und 6 Stunden wurde bei Raumtemperatur gerührt. Danach wurde man die Reaktionsmischung in eiskalte 1 N HCl gegossen, das ausgefallene Rohprodukt wurde abgesaugt und erneut in Essigester gelöst. Nach dem jeweils zweimaligem Waschen mit Wasser, gesättigter Bicarbonatlösung und gesättigter Kochsalzlösung sowie Trocknen der organischen Phase mit Natriumsulfat erhielt man nach dem Einengen zur Trockne 17ß-Cyano-6ß-tosyloxymethyl-18a-homo-19-nor-androst-4-en-3-on, das gleich in der Folgestufe eingesetzt wurde.

Zu einer Lösung von 3 g Trimethylsulfoxoniumiodid in 50 ml trockenem DMSO wurden bei Raumtemperatur portionsweise 450 mg Natriumhydrid gegeben, und e wurde nach beendeter Zugabe 1 Stunde bei Raumtemperatur gerührt. Anschließend wurde die Lösung von 1,5g 17ß-Cyano-6ß-tosyloxymethyl-18a-homo-19-nor-androst-4-en-3-on zum gebildeten Ylid gegeben, und es wurde 6 Stunden bei Raumtemperatur nachgerührt. Nach dem Abbruch der Reaktion durch die Zugabe von 350 ml Wasser, zweimalige Extraktion mit 150 ml Essigester, Wäsche der organischen Phase mit Wasser und gesättigter Kochsalzlösung sowie Trocknen über Natriumsulfat wurde die organische Phase eingeengt, und man erhielt 17ß-Cyano-6,6-ethylen-18a-homo-19-nor-androst-4-en-3-on.
¹H-NMR (300 MHz, CDCl₃ TMS als interner Standard, ausgewählte Signale): δ = 0,40(m,1 H,6,6-ethylen) 0,54(m,1H,6,6-ethylen) 0,68(m,1H, 6,6-ethylen) 0,9-1,13 (m,1H,6,6-ethylen)1,13(m,3H, CH3-CH2) 5,68(s,1H, H-4)

### Beispiel 9

### 17ß-Cyano-6ß,7ß-methylen-18a-homo-19-nor-androst-4-en-3-on und 17ß-Cyano-6α,7α-methylen-18a-homo-19-nor-androst-4-en-3-on

Zu einer Lösung von 3,09 g Trimethylsulfoxoniumiodid in 25 ml trockenem DMSO wurden bei Raumtemperatur portionsweise 468 mg Natriumhydrid gegeben, und es nach wurde nach beendeter Zugabe 1 Stunde bei Raumtemperatur gerührt. Anschließend wurde die Losung von 1,0g 17ß-Cyano-18a-homo-19-nor-androst-4,6-dien-3-on zum gebildeten Ylid gegeben, und es wurde 6 Stunden bei Raumtemperatur nachgerührt. Nach dem Abbruch der Reaktion durch die Zugabe von 150 ml NH₄Cl-Lösung, zweimalige Extraktion mit 75 ml Essigester, Wäsche der organischen Phase mit Wasser und gesättigter Kochsalzlösung sowie Trocknen über Natriumsulfat wurde die organische Phase zur Trockne eingeengt. Flash-Chromatographie an Kieselgel [Hexan / Essigester (0-50%)] lieferte
17ß-Cyano-6ß,7ß-methylen-18a-homo-19-nor-androst-4-en-3-on und
17ß-Cyano-6α,7α-methylen-18a-homo-19-nor-androst-4-en-3-on.

### 17ß-Cyano-6ß,7ß-methylen-18a-homo-19-nor-androst-4-en-3-on:

¹H-NMR (300 MHz, CDCl₃ TMS als interner Standard, ausgewählte Signale): δ = 0,51 (m,1H, 6ß,7ß-methylen) 1,11 (m,3H, CH3-CH2) 6,11 (s,1H, H-4)

### 17ß-Cyano-6α,7α-methylen-18a-homo-19-nor-androst-4-en-3-on:

¹H-NMR (300 MHz, CDCl₃ TMS als interner Standard, ausgewählte Signale): δ = 0,68(m,1H, 6α,7α-methylen) 0.89(m,1H, 6α,7α-methylen) 1,13(m,3H, CH3-CH2) 6,03(s,1H, H-4)

### Beispiel 10

### 17ß-Cyano-17α-methyl-18a-homo-19-nor-androst-4-en-3-on

### 10a.

### 17ß-Cyano-17α-methyl-3-methoxy-18a-homo-19-nor-androst-3,5-dien

Zu einer Lösung von 2,6 g 17ß-Cyano-3-methoxy-18a-homo-19-nor-androst-3,5-dien in 80 ml THF wurden bei -78°C 14,7 ml 2 M Lithiumdüsopropylamidlösung getropft. Es wurde 1 Stunde bei -78°C gerührt, 2,35 ml Methyliodid wurden zugegeben, und es wurde auf Raumtemperatur erwärmen gelassen. 25 ml gesättigte Ammoniumchlorid wurden zugeben, und es wurde mit dreimal 100 ml Essigsäureethylester extrahiert. Die vereinigten organischen Extrakte wurden eingeengt und aus Methanol kristallisiert. Man erhielt 17ß-Cyano-17α-methyl-3-methoxy-18a-homo-19-nor-androst-3,5-dien, welches sofort weiter umgesetzt wurde.

### 10b.

### 17ß-Cyano-17α-methyl-18a-homo-19-nor-androst-4-en-3-on

2g 17ß-Cyano-17α-methyl-3-methoxy-18a-homo-19-nor-androst-3,5-dien wurden in 50 ml Methanol aufgenommen, mit 3 ml 1N Salzsäure versetzt. Nach 1 Stunde wurde mit gesättigter Natriumhydrogencarbonatlösung neutralisiert und im Vakuum eingeengt, wobei das Produkt ausfiel. Es wurde abgesaugt, mit Wasser gewaschen und aus Essisäureethylester umkristallisiert. Man erhielt 17ß-Cyano-17α-methyl-15ß,16ß-methylen-19-nor-androst-4-en-3-on.

### 17ß-Cyano-17α-methyl-15ß,16ß-methylen-19-nor-androst-4-en-3-on:

¹H-NMR (300 MHz, CDCl₃ TMS als interner Standard, ausgewählte Signale): δ = 1,13(m,3H, -CH2-CH3), 1,29(s,3H, 17-CH3), 5,83(s,1H, H-4)

### Beispiel 11

### 17ß-Cyano-17α-ethyl-18a-homo-19-nor-androst-4-en-3-on

### Analog zu den in Beispielen 10a und 10b angegebenen Methoden, wobei Ethyliodid statt Methyliodid verwendet wurde, erhielt man 17ß-Cyano-17α-ethyl-18a-homo-19-nor-androst-4-en-3-on.

### 17ß-Cyano-17α-ethyl-18a-homo-19-nor-androst-4-en-3-on:

¹H-NMR (300 MHz, CDCl₃ TMS als interner Standard, ausgewählte Signale): δ = 1,11(m,6H, CH2-CH3, 17-CH2-CH3), 5,82(s,1H, H-4)

### Beispiel 12

### 17ß-Cyano-15ß,16ß-methylen-18a-homo-19-nor-androst-4-en-3-on

### 12a.

### 17-Cyano-15ß,16ß-methylen-3-methoxy-18a-homo-19-nor-androst-3,5(6)-dien

50 g 15ß,16ß-Methylen-3-methoxy-18a-homo-19-nor-androst-3,5(6)-dien wurden in einem Gemisch aus 860 ml Dimethoxyethan und 603 ml tertiär-Butanol gelöst und portionsweise mit 180 g Kaliumtertiärbutylat versetzt. Anschließend wurden unter starkem Rühren 62,5 g para-Tosylmethylisocyanid (TOSMIC) eingetragen, und es wurde 4 Studen bei Raumtemperatur nachgerührt. Der Ansatz wurde dann auf 1,5 Liter Eiswasser gegossen und mit Ethylacetat extrahiert. Nach Trocknung der organischen Phase über Natriumsulfat wurde filtriert, und das Filtrat wurde eingeengt. Das so erhaltene Rohprodukt wurde ohne Reinigung weiter umgesetzt.

### 12b.

### 17ß-Cyano-15ß,16ß-methylen-18a-homo-19-nor-androst-4-en-3-on und 17α-Cyano-15ß,16ß-methylen-18a-homo-19-nor-androst-4-en-3-on

4,5 g Kieseigei wurden in 7,8 mi Dichlormethan suspendiert und mit 2 ml gesättigter wässriger Oxalsäure versetzt. Anschließend wurden 1,2 g 17-Cyano-15ß,16ß-methylen-3-methoxy-18a-homo-19-nor-androst-3,5(6)-dien, gelöst in 2 ml Dichlormethan, zugegeben, und es wurde 24 Stunden nachgerührt. Dann wurde vom Kieselgel abgesaugt, mit Dichlormethan nachgewaschen und das Filtrat eingeengt. Nach Flashchromatographie an Kieselgel mit einem Gemisch aus Hexan und Ethylacetat wurden die epimeren 17-Nitrile mittels HPLC an chiraler Normalphase mit einem Gemisch aus Hexan und Ethanol getrennt. Als Fraktion I erhielt man 17α-Cyano-15ß,16ß-methylen-18a-homo-19-nor-androst-4-en-3-on und als Fraktion II 17ß-Cyano-15ß,16ß-methylen-18a-homo-19-nor-androst-4-en-3-on.

### 17ß-Cyano-15ß,16ß-methylen-18a-homo-19-nor-androst-4-en-3-on:

¹H-NMR (300 MHz, CDCl₃ TMS als interner Standard, ausgewählte Signale): δ = 0,45(m,1H), 0,90(m,1H), 1,13(t,3H,J=7,3Hz, CH3-CH2), 1,27(m,1H), 1,37(m,1H), 1,67(m,2H), 1,82(m,1H), 1,87(m,1H), 2,06(m,1H), 2,12(m,1H), 2,40(m,2H), 2,68(d breit,1H,J=4,4Hz), 5,86(s,1H, H-4)

### 17α-Cyano-15ß,16ß-methylen-18a-homo-19-nor-androst-4-en-3-on:

¹H-NMR (D6-DMSO) : 0,38(m,1H), 0,72(m,1H), 0,91 (t,3H,J=7,2Hz, CH3-CH2), 2,91 (d breit,1H,J=4,7Hz), 5,71 (s,1H, H-4)

### Beispiel 13

### 17ß-Cyano-15ß,16ß-methylen-18a-homo-19-nor-androst-4,6-dien-3-on

17ß-Cyano-15ß,16ß-methylen-18a-homo-19-nor-androst-4-en-3-on wurde analog zu der in Beispiel 1a angegebenen Vorschrift zum Dienolether umgesetzt, der ohne Reinigung analog zu der in Beispiel 2b angegebenen Vorschrift weiterverarbeitet wurde. Man erhielt 17ß-Cyano-15ß,16ß-methylen-18a-homo-19-nor-androst-4,6-dien-3-on.
¹H-NMR (300 MHz, CDCl₃ TMS als interner Standard, ausgewählte Signale): δ = 0,53(m,1H), 1,07(t,3H,J=7,3Hz, CH3-CH2), 1,84(m,1H), 1,95(m,1H), 2,71(d breit, 1H,J=4,3Hz), 5,81(s,1H, H-4), 6,27(m,1H,H-6), 6,42(m,1H,H-7)

### Beispiel 14

### 17ß-Cyano-6ß,7ß-15ß,16ß-bismethylen-18a-homo-19-nor-androst-4-en-3-on und 17ß-Cyano-6α,7α-15ß,16ß-bismethylen-18a-homo-19-nor-androst-4-en-3-on

17ß-Cyano-15ß,16ß-methylen-18a-homo-19-nor-androst-4,6-dien-3-on wurde analog zu der in Beispiel 9 angegebenen Vorschrift umgesetzt. Nach Aufarbeitung und HPLC-Trennung erhielt man 17ß-Cyano-6ß,7ß-15ß,16ß-bismethylen-18a-homo-19-nor-androst-4-en-3-on und 17ß-Cyano-6α,7α-15ß,16ß-bismethylen-18a-homo-19-nor-androst-4-en-3-on.

### 17ß-Cyano-6ß,7ß-15ß,16ß-bismethylen-18a-homo-19-nor-androst-4-en-3-on:

¹H-NMR (300 MHz, CDCl₃ TMS als interner Standard, ausgewählte Signale): δ = 0,51 (m,1H), 0,59(m,1H), 1,03(t,3H,J=7,3Hz, CH3-CH2), 1,20(m,1H), 1,31 (m,1H), 1,73(m,2H), 2,09(m,1H), 2,15(m,1H), 2,20(m,1H), 2,28(m,1H), 2,44(m,2H), 2,70(d breit,1H,J=4,4Hz), 6,13(s,1H, H-4)

### 17ß-Cyano-6α,7α-15ß,16ß-bismethylen-18a-homo-19-nor-androst-4-en-3-on:

¹H-NMR (300 MHz, CDCl₃ TMS als interner Standard, ausgewählte Signale): δ = 0,48(m,1H), 0,79(m,1H), 0,84(m,1H), 1,05(t,3H,J=7,3Hz, CH3-CH2), 1,16(m,1H), 1,41(m,1H), 2,68(d breit,1H,J=4,4Hz), 6,05(s,1H, H-4)

### Beispiel 15

### 17ß-Cyano-17α-methyl-15ß,16ß-methylen-18a-homo-19-nor-androst-4-en-3-on

Aus 17-Cyano-15ß,16ß-methylen-3-methoxy-18a-homo-19-nor-androst-3,5(6)-dien erhielt man nach den in den Beispielen 10a und 10b angegebenen Vorschriften 17ß-Cyano-17α-methyl-15ß,16ß-methylen-18a-homo-19-nor-androst-4-en-3-on.
¹H-NMR (300 MHz, CDCl₃ TMS als interner Standard, ausgewählte Signale): δ = 0,42 (m,1H), 0,88(m,1H), 1,04(t,3H,J=7,3Hz, CH3-CH2), 1,37(s,3H), 5,86(s,1H, H-4)

### Beispiel 16

### 17ß-Cyano-17α-methyl-15ß,16ß-methylen-18a-homo-19-nor-androsta-4,6-dien-3-on

Analog zu Beispiel 2b wurde aus 17ß-Cyano-17α-methyl-15ß,16ß-methylen-3-meth-oxy-18a-homo-19-nor-androst-3,5-dien 17ß-Cyano-17α-methyl-15ß,16ß-methylen-18a-homo-19-nor-androsta-4,6-dien-3-on erhalten.
¹H-NMR (300 MHz, CDCl₃ TMS als interner Standard, ausgewählte Signale): δ = 0,53(m,1H, cyclopropyl), 1,10(m,3H, CH2-CH3), 1,43(s,1H, 17-CH3), 5,84(s,1H, H-4), 6,30(m,1H, H-6), 6,46(m,1H,H-7)

### Beispiel 17

### 17ß-Cyano-7α,17α-bismethyl-18a-homo-19-nor-androst-4-en-3-on

Aus 17ß-Cyano-17α-methyl-18a-homo-19-nor-androsta-4,6-dien-3-on erhält man analog der in Beispiel 3 angegeben Vorschrift nach HPLC-Trennung 17ß-Cyano-7α17α-bismethyl-18a-homo-19-nor-androst-4-en-3-on .

### 17ß-Cyano-7α,17α-bismethyl-18a-homo-19-nor-androst-4-en-3-on:

¹H-NMR (CDCl₃): 0.88 (d,3H,J=7.34Hz, 7-CH3), 1.05(m,3H, CH2-CH3 ),1.39(s,3H,17-CH3), 5.85(s,1H, H-4)

### Beispiel 18

### 17ß-Cyano-7α-ethyl-17α-methyl-18a-homo-19-nor-androst-4-en-3-on und 17ß-Cyano-7ß-ethyl-17α-methyl-18a-homo-19-nor-androst-4-en-3-on

Aus 17ß-Cyano-17α-methyl-15ß,16ß-methylen-18a-homo-19-nor-androsta-4,6-dien-3-on erhält man analog der in Beispiel 3 angegeben Vorschrift unter Verwendung von Ethylmagnesiumbromid statt Methylmagnesiumbromid nach HPLC-Trennung17ß-Cyano-7α-ethyl-17α-methyl-18a-homo-19-nor-androst-4-en-3-on und 17ß-Cyano-7ß-ethyl-17α-methyl-18a-homo-19-nor-androst-4-en-3-on

### 17ß-Cyano-7α-ethyl-17α-methyl -18a-homo-19-nor-androst-4-en-3-on:

¹H-NMR (CDCl₃):0.92 (m,3H, 7-CH2-CH3), 1.04(m,3H, CH2-CH3), 1.38(s,3H,17-CH3),5.87(s,1H, H-4)

### 17ß-Cyano-7ß-ethyl-17α-methyl 18a-homo-19-nor-androst-4-en-3-on:

¹H-NMR (CDCl₃): 0.92 (m,3H, 7-CH2-CH3), 1.04(m,3H, CH2-CH3), 1.39(s,3H,17-CH3),5.84(s,1H, H-4)

### Beispiel 19

### 17ß-Cyano-17α-methyl-7α-vinyl-18a-homo-19-nor-androst-4-en-3-on und 17ß-Cyano-17α-methyl-7ß-vinyl-18a-homo-19-nor-androst-4-en-3-on

Aus 17ß-Cyano-17α-methyl-15ß,16ß-methylen-18a-homo-19-nor-androsta-4,6-dien-3-on erhält man analog der in Beispiel 3 angegeben Vorschrift unter Verwendung von Vinylmagnesiumbromid statt Methylmagnesiumbromid nach HPLC-Trennung17ß-Cyano-17α-methyl-7α-vinyl-18a-homo-19-nor-androst-4-en-3-on und 17ß-Cyano-17α-methyl-7ß-vinyl-18a-homo-19-nor-androst-4-en-3-on.

### 17ß-Cyano-17α-methyl-7α-vinyl-18a-homo-19-nor-androst-4-en-3-on:

¹H-NMR (CDCl₃): 1.05(m,3H, CH2-CH3),1.36(s,3H,17-CH3),5.17(m,2H, CH2=CH), 5.83 (m,1H,CH2=CH) 5.87(s,1H, H-4)

### 17ß-Cyano-17α-methyl-7ß-vinyl-18a-homo-19-nor-androst-4-en-3-on:

¹H-NMR (CDCl₃): 1.05(m,3H, CH2-CH3),1.35(s,3H,17-CH3),5.02(m,2H, CH2=CH), 5.90 (m,1H,CH2=CH) 5.85(s,1H, H-4)

### Beispiel 20

### 17ß-Cyano-7α-cyclopropyl-17α-methyl-18a-homo-19-nor-androst-4-en-3-on und 17ß-Cyano-7ß-cyclopropyl-17α-methyl-18a-homo-19-nor-androst-4-en-3-on

Aus 17ß-Cyano-17α-methyl-18a-homo-19-nor-androsta-4,6-dien-3-on erhält man analog der in Beispiel 3 angegeben Vorschrift unter Verwendung von Cyclopropylmagnesiumbromid statt Methylmagnesiumbromid nach HPLC-Trennung 17ß-Cyano-7□-cyclopropyl-17α-methyl-18a-homo-19-nor-androst-4-en-3-on und 17ß-Cyano-7ß-cyclopropyl-17α-methyl-18a-homo-19-nor-androst-4-en-3-on.

### 17ß-Cyano-17α-methyl-7α-cyclopropyl-18a-homo-19-nor-androst-4-en-3-on:

¹H-NMR (CDCl₃): 0.05(m,1H,cyclopropyl), 0.35(m,1H,cyclopropyl), 0.49(m,1H, cyclopropyl),0.59(m,2H,cyclopropyl) 1.06(m,3H, CH2-CH3),1.40(s,3H,17-CH3) 5.90(s,1H, H-4)

### 17ß-Cyano-17α-methyl-7ß-cyclopropyl-18a-homo-19-nor-androst-4-en-3-on:

¹H-NMR (CDCl₃): 0.22-0.90(m,cyclopropyl), 1.07(m,3H, CH2-CH3),1.38(s,3H,17-CH3) 5.82(s,1H, H-4)

### Beispiel 21

### 17ß-Cyano-17α-methyl-6α,7α-methylen-18a-homo-19-nor-androst-4-en-3-on und 17ß-Cyano-17α-methyl-6ß,7ß-methylen-18a-homo-19-nor-androst-4-en-3-on

Aus 17ß-Cyano-17α-methyl-18a-homo-19-nor-androsta-4,6-dien-3-on erhält man analog der in Beispiel 9 angegeben Vorschrift 17ß-Cyano-17α-methyl-6α,7α-methylen-18a-homc-19-nor-androst-4-en-3-on und 17ß-Cyano-17α-methyl-6ß,7ß-methylen-18a-homo-19-nor-androst-4-en-3-on.

### 17ß-Cyano-17α-methyl-6ß,7ß-methylen-18a-homo-19-nor-androst-4-en-3-on:

¹H-NMR (CDCl₃): 0.49(m,1H, 6ß,7ß-methylen),0.59(m,1H, 6ß,7ß-methylen) 1.02(m,3H, CH2-CH3),1.40(s,3H,17-CH3), 6.12(s,1H, H-4)

### 17ß-Cyano-17α-methyl-6α,7α-methylen-18a-homo-19-nor-androst-4-en-3-on:

¹H-NMR (CDCl₃): 0.46(m,1H, 6□,7□-methylen), 1.04(m,3H, CH2-CH3),1.39(s,3H,17-CH3), 6.05(m,1H, H-4)

### Beispiel 22

### 17ß-Cyano-17α-ethyl-15ß,16ß-methylen-18a-homo-19-nor-androst-4-en-3-on

### 22a.

### 17ß-Cyano-17α-ethyl-15ß,16ß-methylen-3-methoxy-18a-homo-19-nor-androst-3,5(6)-dien

Aus 17-Cyano-15ß, 16ß-methylen-3-methoxy-18a-homo-19-nor-androst-3,5(6)-dien erhielt man analog zu der in Beispiel 10a angegebenen Vorschrift, wobei das dort verwendete Methyliodid gegen Ethyliodid ausgetauscht wurde, 17ß-Cyano-17α-ethyl-15ß,16ß-methylen-3-methoxy-18a-homo-19-nor-androst-3,5(6)-dien.
¹H-NMR (d6-DMSO) : 0,39(m,1H), 0,94(t,3H,J=7,3Hz), 1,12(t,3H,J=7,3Hz,), 3,49(s,3H,-3-O-CH3), 5,25(s,1H, H-4), 5,31 (s breit,1H, H-6)

### 22b.

### 17ß-Cyano-17α-ethyl-15ß,16ß-methylen-18a-homo-19-nor-androst-4-en-3-on

17ß-Cyano-17α-ethyl-15ß,16ß-methylen-3-methoxy-18a-homo-19-nor-androst-3,5(6)-dien wurde analog zu der in Beispiel 10b angegebenen Vorschrift umgesetzt. Man erhielt 17ß-Cyano-17α-methyl-15ß,16ß-methylen-18a-homo-19-nor-androst-4-en-3-on.
¹H-NMR (300 MHz, CDCl₃ TMS als interner Standard, ausgewählte Signale): δ = 0,42(m,1H), 0,88(m,1H), 1,04(t,3H,J=7,3Hz, CH3-CH2), 1,37(s,3H), 5,86(s,1H, H-4)

### Beispiel 23

### 17ß-Cyano-17α-ethyl-15ß,16ß-methylen-18a-homo-19-nor-androsta-4,6-dien-3-on

Analog zu Beispiel 2b wurde aus 17ß-Cyano-17α-ethyl-15ß,16ß-methylen-3-meth-oxy-18a-homo-19-nor-androst-3,5-dien 17ß-Cyano-17α-ethyl-15ß,16ß-methylen-18a-homo-19-nor-androsta-4,6-dien-3-on erhalten.
¹H-NMR (d6-DMSO): 0,43(m,1H, cyclopropyl), 0,92(t,3H,J=7,3Hz), 1,08(t,3H,J=7,3Hz), 5,72(s,1H, H-4), 6,27(m,1H, H-6), 6,46(m,1H,H-7)

### Beispiel 24

### 17β-Cyano-17α-ethyl-7α-methyl15β,16β-methylen-18a-homo-19-nor-androst-4-en-3-on und 17β-Cyano-17α-ethyl-7β-methyl15β,16β-methylen-18a-homo-19-nor-androst-4. en-3-on

Aus 17β-Cyano-17α-ethyl-15β, 16β-methylen-18a-homo-19-nor-androsta-4,6-dien-3-on erhielt man analog zu der in Beispiel 3 angegebenen Vorschrift nach HPLC-Trennung 17β-Cyano-17a-ethyl-7α-methyl-15β, 16β-methylen-18a-homo-19-nor-androst-4-en-3-on und 17β-Cyano-17α-ethyl-7β-methyl-15β,16β-methylen-18a-homo-19-nor-androst-4-en-3-on.

### 17β-Cyano-17α-ethyl-7α-methyl-15β,16β-methylen-18a-homo-19-nor-androst-4-en-3-on:

¹H-NMR (CDCl₃): 0.44(m,1H, Cyclopropyl), 0.87 (d, 3H, J=7.0, 7-CH₃), 1.05 (t, 3H, J=7.3, -CH₂-CH₃), 1.22 (t, 3H, J=7.3, -CH₂-CH₃), 1.33 (m, 1H), 1.75 (m, 1H), 1.81 (m, 1H), 2.08 (m, 1H), 2.42 (m, 1H), 2.57 (m, 1H), 5.87 (s, 1H, H-4)

### 17β-Cyano-17α-ethyl-7β-methyl-15β, 16β-methylen-18a-homo-19-nor-androst-4-en-3-on:

¹H-NMR (CDCl₃): 0.51 (m,1H, Cyclopropyl), 2.18-2.31 (m, 2H), 2.38 (m, 1), 2.48 (m, -1 H); 5.82 (s, 1H, H-4)

### Beispiel 25

### 17β-Cyano-7α,17α-bisethyl-15β,16β-methylen-18a-homo-19-nor-androst-4-en-3-on und 17β-Cyano-7β,17α-bisethyl-15β,16β-methylen-18a-homo-19-nor-androst-4-en-3-on

Aus 17β-Cyano-17a-ethyl-15β,16β-methylen-18a-homo-19-nor-androsta-4,6-dien-3-on erhielt man analog zu der in Beispiel 3 angegebenen Vorschrift unter Verwendung von Ethylmagnesiumbromid statt Methylmagnesiumbromid nach HPLC-Trennung 17β-Cyano-7α, 17α-bisethyl-15β,16β-methylen-18a-homo-19-nor-androst-4-en-3-on und 17β-Cyano-7β,17α-bisethyl-15β,16β-methylen-18a-homo-19-nor-androst-4-en-3-on.

### 17β-Cyano-7α-,17α-bisethyl-15β, 16β-methylen-18a-homo-19-nor-androst-4-en-3-on:

¹H-NMR (CDCl₃): 0.45 (m,1H, Cyclopropyl), 0.92 (t, 3H, J=7.34, -CH₂-CH₃), 1.04 (t, 3H, J=7.34, -CH₂-CH₃), 1.21 (t, 3H, J=7.3, -CH₂-CH₃), 1.40 (m, 2H), 2.62 (m, 1H), 5.87 (s, 1H, H-4)

### 17β-Cyano-7β,17α-bisethyl-15β, 16β-methylen-18a-homo-19-nor-androst-4-en-3-on:

¹H-NMR (CDCl₃): 0.51 (m,1H, Cyclopropyl), 0.92 (t, 3H, J=7.34, -CH₂-CH₃), 1.03 (t, 3H, J=7.34, -CH₂-CH₃), 1.20 (t, 3H, J=7.3, -CH₂-CH₃), 2.61 (m, 1H), 5.84 (s, 1H, H-4)

### Beispiel 26

### 17β-Cyano-17α-ethyl-15β,16β-methylen-7a-vinyl-18a-homo-19-nor-androst-4-en-3-on und 17β-Cyano-17α-ethyl-15β,16β-methylen-7β-vinyl-18a-homo-19-nor-androst-4-en-3-on

Aus 17β-Cyano-17α-ethyl-15β,16β-methylen-18a-homo-19-nor-androsta-4,6-dien-3-on erhielt man analog zu der in Beispiel 3 angegebenen Vorschrift unter Verwendung von Vinylmagnesiumbromid statt Methylmagnesiumbromid nach HPLC-Trennung 17β-Cyano-17α-ethyl-15β,16β-methylen-7α-vinyl-18a-homo-19-nor-androst-4-en-3-on und 17β-Cyano-17α-ethyl-15β, 16β-methylen-7β-vinyl-18a-homo-19-nor-androst-4-en-3-on.

### 17β-Cyano-17α-ethyl-15β,16β-methylen-7α-vinyl-18a-homo-19-nor-androst-4-en-3-on:

¹H-NMR (CDCl₃): 0.45 (m,1H, Cyclopropyl), 1.05 (t, 3H, J=7.28, -CH₂-CH₃), 1.20 (t, 3H, J=7.28, -CH₂-CH₃), 2.13 (m, 1H), 2.28 (m, 2H), 2.43 (m, 1H), 2.52 (m, 1H), 2.64 (m, 1H), 2.78 (m, 1H), 5.14 (m , 1H, CH2=CH), 5.18 (m , 1H, CH2=CH), 5.82 (m, 1H, CH2=CH), 5.87 (s, 1H, H-4)

### 17β-Cyano-17α-ethyl-15β,16β-methylen-7β-vinyl-18a-homo-19-nor-androst-4-en-3-on:

¹H-NMR (CDCl₃): 0.40 (m,1H, Cyclopropyl), 1.04 (t, 3H, J=7.28, -CH₂-CH₃), 1.18 (t, 3H, J=7.28, -CH₂-CH₃), 4.98 (m , 1H, CH2=CH), 5.05 (m , 1H, CH2=CH), 5.85 (s, 1H, H-4), 5.90 (m, 1H, CH2=CH)

### Beispiel 27

### 17β-Cyano-7α-cyclopropyl-17α-ethyl-15β,16β-methylen-18a-homo-19-nor-androst-4-en-3-on und 17β-Cyano-7β-cyclopropyl-17a-ethyl-15β,16β-methylen-18a-homo-19-nor-androst-4-en-3-on

Aus 17β-Cyano-17α-ethyl-15β,16β-methylen-18a-homo-19-nor-androsta-4,6-dien-3-on erhielt man analog zu der in Beispiel 3 angegebenen Vorschrift unter Verwendung von Cyclopropylmagnesiumbromid statt Methylmagnesiumbromid nach HPLC-Trennung 17β-Cyano-7α-cyclopropyl-17α-ethyl-15β,16β-methylen-18a-homo-19-nor-androst-4-en-3-on und 17β-Cyano-7β-cyclopropyl-17α-ethyl-15β,16β-methylen-18a-homo-19-nor-androst-4-en-3-on.

### 17β-Cyano-7α-cyclopropyl-17α-ethyl-15β,16β-methylen-18a-homo-19-nor-androst-4-en-3-on:

¹H-NMR (CDCl₃): 0.06 (m, 1H), 0.34 (m, 1H), 0.42 (m, 1H), 0.48 (m, 1H), 0.58 (m, -2H), 1.06 (t, 3H, J=7.28, CH2-CH3); 1.23 (t, 3H, J=7.28, -CH₂-CH₃), 1.89 (m, 1H), 1.97 (m, 2H), 5.90 (s, 1 h, H-4)

### 17β-Cyano-7β-cyclopropyl-17α-ethyl-15β,16β-methylen-18a-homo-19-nor-androst-4-en-3-on:

¹'H-NMR (CDCl₃): 0.28 (m, 2H), 0.45 (m, 1H), 0.59 (m, 2H), 0.79 (m, 1H), 0.92 (m, 1H), 1.06 (t, 3H, J=7.28, -CH₂-CH₃), 1.21 (t, 3H, J=7.28, -CH₂-CH₃), 2.40 (m, 1H), 2.56 (m, 1H), 5.90 (s, 1H, H-4)

### Beispiel 28

### 17β-Cyano-17α-ethyl-6α,7α-15β,16β-bismethylen-18a-homo-19-nor-androst-4-en-3-on und 17β-Cyano-17α-ethyl-6β,7β-15β,16β-bismethylen-18a-homo-19-nor-androst-4-en-3-on

Aus17β-Cyano-17α-ethyl-15β,16β-methylen-18a-homo-19-nor-androsta-4,6-dien-3-on erhielt man analog zu der in Beispiel 9 angegebenen Vorschrift nach HPLC-Trennung 17β-Cyano-17α-ethyl-6α,7α-15β,16β-bismethylen-18a-homo-19-nor-androst-4-en-3-on und 17β-Cyano-17α-ethyl-6β,7β-15β,16β-bismethylen-18a-homo-19-nor-androst-4-en-3-on.

### 17β-Cyano-17α-ethyl-6α,7α-15β,16β-bismethylen-18a-homo-19-nor-androst-4-en-3-on:

¹H-NMR (CDCl₃): 0.47 (m, 1H), 0.77 (m, 1H), 0.81 (m, 1H), 1.04 (t, 3H, J=7.15, -CH₂-CH₃), 1.21 (t, 3H, J=7.33, -CH₂-CH₃), 2.27 (m, 1H), 2.50 (m, 1H), 6.04 (s, 1H, H-4)

### 17β-Cyano-17α-ethyl-6β,7β-15β,16β-bismethylen-18a-homo-19-nor-androst-4-en-3-on:

¹H-NMR (CDCl₃): 0.50 (m, 1H), 0.59 (m, 1H), 1.02 (t, 3H, J=7.34, -CH₂-CH₃), 1.22 (t, 3H, J=7.34, -CH₂-CH₃), 1.31 (m, 1H), 2.16 (m, 2H), 2.29 (m, 1H), 2.43 (m, 1H), 6.12 (s, 1H, H-4)

### Beispiel 29

### 17β-Cyano-7α,17α-bismethyl-15β,16β-methylen -18a-homo-19-nor-androst-4-en-3-on

Aus 17β-Cyano-17α-methyl-**15β,16β-methylen** -18a-homo-19-nor-androsta-4,6-dien-3-on erhält man analog der in Beispiel 3 angegeben Vorschrift nach HPLC-Trennung 17β-Cyano-7α,17α-bismethyl-**15β,16β-methylen** -18a-homo-19-nor-androst-4-en-3-on .

### 17β-Cyano-7α,17α-bismethyl-15β,16β-methylen -18a-homo-19-nor-androst-4-en-3-on:

¹H-NMR (CDCl₃ ): 0.44 (m, 1H, cyclopropyl), 0.89 (d,3H,J=7.34Hz, 7-CH3), 1.05(m,3H, CH2-CH3),1.39(s,3H,17-CH3), 5.87(s,1H, H-4)

### Beispiel 30

### 17β-Cyano-7α-ethyl-17α-methyl-15β,16β-methylen -18a-homo-19-nor-androst-4-en-3-on und 17β-Cyano-7β-ethyl-17α-methyl-15β,16β-methylen -18a-homo-19-nor-androst-4-en-3-on

Aus 17β-Cyano-17α-methyl-15β,16β-methylen-18a-homo-19-nor-androsta-4,6-dien-3-on erhält man analog der in Beispiel 3 angegeben Vorschrift unter Verwendung von Ethylmagnesiumbromid statt Methylmagnesiumbromid nach HPLC-Trennung 17β-Cyano-7α-ethyl-17α-methyl-15β,16β-methylen-18a-homo-19-nor-androst-4-en-3-on und 17β-Cyano-7β-ethyl-17α-methyl-15β, 16β-methylen-18a-homo-19-nor-androst-4-en-3-on

### 17β-Cyano-7α-ethyl-17α-methyl -15β,16β-methylen-18a-homo-19-nor-androst-4-en-3-on:

¹H-NMR (CDCl₃):0.44 (m,1H, cyclopropyl) 0.93 (m,3H, 7-CH2-CH3), 1.04(m,3H, CH2-CH3), 1.38(s,3H,17-CH3),5.87(s,1H, H-4)

### 17β-Cyano-7β-ethyl-17α-methyl-15β,16β-methylen-18a-homo-19-nor-androst-4-en-3-on:

¹H-NMR (CDCl₃ ): 0.51 (m, 1H, cyclopropyl)0.94 (m,3H, 7-CH2-CH3), 1.05(m,3H, CH2-CH3),1.37(s,3H,17-CH3),5.85(s,1H, H-4)

### Beispiel 31

### 17β-Cyano-17α-methyl--7α-vinyl-15β,16β-methylen-18a-homo-19-nor-androst-4-en-3-on und 17β-Cyano-17α-methyl-7β-vinyl-15β,16β-methylen-18a-homo-19-nor-androst-4-en-3-on

Aus 17β-Cyano-17α-methyl-15β,16β-methylen-18a-homo-19-nor-androsta-4,6-dien-3-on erhält man analog der in Beispiel 3 angegeben Vorschrift unter Verwendung von Vinylmagnesiumbromid statt Methylmagnesiumbromid nach HPLC-Trennung 7β-Cyano-17α-methyl-7α-vinyl-15β,16β-methylen-18a-homo-19-nor-androst-4-en-3-on und 17β-Cyano-17α-methyl-7β-vinyl-15β,16β-methylen-18a-homo-19-nor-androst-4-en-3-on.

### 17β-Cyano-17α-methyl-7α-vinyl-15β, 16β-methylen-18a-homo-19-nor-androst-4-en-3-on:

¹H-NMR (CDCl₃ ): 0.44 (m,1H, cyclopropyl) 1.05(m,3H, CH2-CH3),1.37(s,3H,17-CH3),5.17(m,2H, CH2=CH), 5.83 (m, 1H,CH2=CH) 5.88(s,1H, H-4)

### 17β-Cyano-17α-methyl-7β-vinyl-15β,16β-methylen-18a-homo-19-nor-androst-4-en-3-on:

¹H-NMR (CDCl₃): 0.40 (m,1H, cyclopropyl) 1.06(m,3H, CH2-CH3),1.36(s,3H,17-CH3),5.03(m,2H, CH2=CH), 5.90 (m,1H,CH2=CH) 5.86(s,1H, H-4)

### Beispiel 32

### 17β-Cyano-7α-cyclopropyl-17α-methyl-15β,16β-methylen-18a-homo-19-nor-androst-4-en-3-on und 17β-Cyano-7β-cyclopropyl-17α-methyl-15β,16β-methylen-18a-homo-19-nor-androst-4-en-3-on

Aus 17β-Cyano-17α-methyl-15β,16β-methylen-18a-homo-19-nor-androsta-4,6-dien-3-on erhält man analog der in Beispiel 3 angegeben Vorschrift unter Verwendung von Cyclopropylmagnesiumbromid statt Methylmaqnesiumbromid nach HPLC-Trennung 17β-Cyano-7α-cyclopropyl-17α-methyl-15β, 16β-methylen-18a-homo-19-nor-androst-4-en-3-on und 17β-Cyano-7β-cyclopropyl-17α-methyl-15β,16β-methylen-18a-homo-19-nor-androst-4-en-3-on.

### 17β-Cyano-17α-methyl-7α-cyclopropyl-15β,16β-methylen-18a-homo-19-nor-androst-4-en-3-on:

¹H-NMR (CDCl₃): 0.07(m,1H,cyclopropyl), 0.35(m,1H,cyclopropyl), 0.41(m,1H,cyclopropyl), 0.50(m,1H, cyclopropyl),0.59(m,2H,cyclopropyl) 1.07(m,3H, CH2-CH3),1.40(s,3H,17-CH3) 5.90(s,1H, H-4)

### 17β-Cyano-17α-methyl-7β-cyclopropyl-15β,16β-methylen-18a-homo-19-nor-androst-4-en-3-on:

¹H-NMR (CDCl₃ ): 0.22-0.90(m,cyclopropyl), 1.06(m,3H, CH2-CH3),1.38(s,3H,17-CH3) 5.82(s,1H, H-4)

### Beispiel 33

### 17β-Cyano-17α-methyl-6α,7α-methylen-15β,16β-methylen-18a-homo-19-norandrost-4-en-3-on und 17β-Cyano-17α-methyl-6β,7β-methylen-15β,16β-methylen-18a-homo-19-nor-androst-4-en-3-on

Aus 17β-Cyano-17α-methyl-15β, 16β-methylen-18a-homo-19-nor-androsta-4,6-dien-3-on erhält man analog der in Beispiel 9 angegeben Vorschrift 17β-Cyano-17a-methyl-6α,7α-methylen-15β, 16β-methylen-18a-homo-19-nor-androst-4-en-3-on und 17β-Cyano-17α-methyl-6β,7β-methylen-15β,16β-methylen-18a-homo-19-nor-androst-4-en-3-on.

### 17β-Cyano-17α-methyl-6α,7α-methylen-15β,16β-methylen-18a-homo-19-norandrost-4-en-3-on:

¹H-NMR (CDCl₃): 0.47(m,1H, 6α,7α-methylen), 1.05(m,3H, CH2-CH3),1.40(s,3H,17-CH3), 6.06(m,1 H, H-4)

### 17β-Cyano-17α-methyl-6β,7β-methylen-15β,16β-methylen-18a-homo-19-norandrost-4-en-3-on:

¹H-NMR (CDCl₃): 0.49(m,1H, 6β,7β-methylen),0.60(m,1H, 6β,7β-methylen) 1.03(m,3H, CH2-CH3),1.40(s,3H,17-CH3), 6.13(s,1H, H-4)

### Beispiel 34

### 4-Chlor-17β-cyano-17α-methyl-15β,16β-methylen-18a-homo-19-nor-androst-4-en-3-on

100 mg 17β-cyano-17α-methyl-15β, 16β-methylen-18a-homo-19-nor-androst-4-en-3-on werden in 1 ml Pyridin gelöst und auf 0°C gekühlt. Nach Zusatz von 42 µl Sulfurylchlorid wird 1,5 Stunden bei dieser Temperatur nachgerührt.
Nach Versetzten mit gesättigter wässriger Natriumhydrogencarbonatlösung, Wasser und Ethylacetat werden die Phasen getrennt und die organische Phase mit Wasser und gesättigter wässsriger Natriumchloridlösung gewaschen. Nach Trocknung der organischen Phase über Natriumsulfat und Filtration wird eingeengt. Man erhält 4-Chlor-17β-cyano-17□-methyl-15β,16β-methylen-18a-homo-19-nor-androst-4-en-3-on.

### 4-Chlor-17β-cyano-17□-methyl-15β,16β-methylen-18a-homo-19-nor-androst-4-en-3-on:

¹H-NMR (CDCl₃): 0.44 (m, 1H), 1.05 (t, 3H, J=7.35, -CH₂-CH₃), 3.43 (m, 1H)

### Beispiel 35

### 17β-Cyano-3-hydroxyimino-17α-methyl-15β,16β-methylen-18a-homo-19-norandrost-4-en

100 mg 17β-Cyano-17α-methyl-15β,16β-methylen-18a-homo-19-nor-androst-4-en-3-on werden in 1 ml Pyridin gelöst und mit 34,5 mg Hydroxylaminhydrochlorid versetzt. Nach einstündigem Rühren bei 125°C Badtemperatur wird der Ansatz zwischen Wasser und Ethylacetat verteilt. Die organische Phase wird mit Wasser und gesättigter wässriger Natriumchloridlösung gewaschen, über Natriumsulfat getrocknet, filtriert und eingeengt. Es wird 17β-Cyano-3-hydroxyimino-17α-methyl-15β,16β-methylen-18a-homo-19-nor-androst-4-en als E/Z-Gemisch der Oxime erhalten.

### 17β-Cyano-3-hydroxyimino-17α-methyl-15β,16β-methylen-18a-homo-19-norandrost-4-en:

¹H-NMR (CDCl₃): 0.41 (m, 1H), 1.03 (t, 3H, J=7.35, -CH₂-CH₃), 1,36 (s, 3H, -CH₃), 5.91 und 6.58 (jeweils s, zusammen 1H, H-4)

## Patentansprüche

1. 17β-Cyano-18a-homo-19-nor-androst-4-en-Derivat mit der allgemeinen chemischen Formel 1 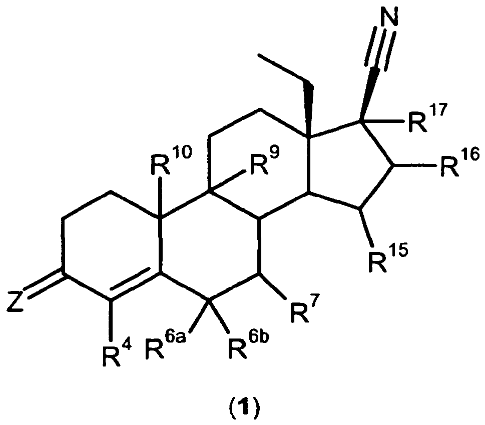 wobei
Z⁻ ausgewählt ist aus der Gruppe, umfassend O, zwei Wasserstoffatome, NOR und NNHSO₂R, worin R Wasserstoff oder C₁-C₄-Alkyl ist,
R⁴ Wasserstoff oder Halogen ist,
ferner entweder:
R^{6a}, R^{6b} gemeinsam Methylen oder 1,2-Ethandiyl bilden oder R^{6a} Wasserstoff ist und R^{6b} aus der Gruppe ausgewählt ist, umfassend Wasserstoff, Methyl und Hydroxymethylen, und
R⁷ ausgewählt ist aus der Gruppe, umfassend Wasserstoff, C₁-C₄-Alkyl, C₂-C₃-Hikenyi und Cyclopropyl,
oder:
R^{6a} Wasserstoff ist und R^{6b} und R⁷ entweder gemeinsam Methylen bilden oder unter Bildung einer Doppelbindung zwischen C⁶ und C⁷ entfallen,
R⁹, R¹⁰ Wasserstoff sind oder unter Bildung einer Doppelbindung zwischen C⁹ und C¹⁰ entfallen,
R¹⁵, R¹⁶ Wasserstoff sind oder gemeinsam Methylen bilden,
R¹⁷ ausgewählt ist aus der Gruppe, umfassend Wasserstoff, C₁-C₄-Alkyl und Allyl,
wobei mindestens einer der Substituenten R⁴, R^{6a}, R^{6b}, R⁷, R¹⁵, R¹⁶ und R¹⁷ ungleich Wasserstoff ist oder R^{6b} und R⁷ unter Bildung einer Doppelbindung zwischen C⁶ und C⁷ entfallen,
sowie deren Solvate, Hydrate und Salze, worin die Reste R^{6a}, R^{6b}, R⁷, R¹⁵ und R¹⁶ sowie, falls vorhanden, die aus R^{6b} und R⁷ gebildete Methylengruppe und, falls vorhanden, die aus R¹⁵ und R¹⁶, gebildete Methylengruppe sowohl α- oder β-ständig sein können und C₁₋₄-Alkylgruppen perfluoriert sein konnen.

2. 17β-Cyano-18a-homo-19-nor-androst-4-en-Derivat nach Anspruch 1, **dadurch gekennzeichnet, dass** R¹⁵ und R¹⁶ gemeinsam Methylen bilden.

3. 17β-Cyano-18a-homo-19-nor-androst-4-en-Derivat nach einem der vorstehenden Ansprüche, **dadurch gekennzeichnet, dass** Z ausgewählt ist aus der Gruppe, umfassend O, NOH und NNHSO₂H.

4. 17β-Cyano-18a-homo-19-nor-androst-4-en-Derivat nach einem der vorstehenden Ansprüche, **dadurch gekennzeichnet, dass** Z für O steht.

5. 17β-Cyano-18a-homo-19-nor-androst-4-en-Derivat nach einem der vorstehenden Ansprüche, **dadurch gekennzeichnet, dass** R⁴ Wasserstoff oder Chlor ist.

6. 17β-Cyano-18a-homo-19-nor-androst-4-en-Derivat nach einem der vorstehenden Ansprüche, **dadurch gekennzeichnet, dass** R^{6a}, R^{6b} gemeinsam 1,2-Ethandiyl bilden oder jeweils Wasserstoff sind.

7. 17β-Cyano-18a-homo-19-nor-androst-4-en-Derivat nach einem der vorstehenden Ansprüche, **dadurch gekennzeichnet, dass** R⁷ ausgewählt ist aus der Gruppe, umfassend Wasserstoff und Methyl.

8. 17β-Cyano-18a-homo-19-nor-androst-4-en-Derivat nach einem der Ansprüche 1 bis 5, **dadurch gekennzeichnet, dass** R^{6b} und R⁷ gemeinsam Methylen bilden.

9. 17β-Cyano-18a-homo-19-nor-androst-4-en-Derivat nach einem der vorstehenden Ansprüche, **dadurch gekennzeichnet, dass** R¹⁷ ausgewählt ist aus der Gruppe, umfassend Wasserstoff und Methyl.

10. 17β-Cyano-18a-homo-19-nor-androst-4-en-Derivat nach Anspruch 1, ausgewählt aus der Gruppe, umfassend
17β-Cyano-17α-methyl-15β,16β-methylen-18a-homo-19-nor-androst-4-en-3-on,
17β-Cyano-15β,16β-methylen-18a-homo-19-nor-androst-4-en-3-on,
17β-Cyano-6β-hydroxymethylen-18a-homo-19-nor-androst-4-en-3-on,
17β-Cyano-6,6-ethandiyl-18a-homo-19-nor-androst-4-en-3-on,
17β-Cyano-6β,7β-methylen-18a-homo-19-nor-androst-4-en-3-on,
17β-Cyano-6a,7a-methylen-18a-homo-19-nor-androst-4-en-3-on,
17β-Cyano-17a-methyl-18a-homo-19-nor-androst-4-en-3-on,
17β-Cyano-17a-ethyl-18a-homo-19-nor-androst-4-en-3-on,
17β-Cyano-7a-methyl-18a-homo-19-nor-androst-4-en-3-on,
17β-Cyano-7β-ethyl-18a-homo-19-nor-androst-4-en-3-on,
17β-Cyano-7α-ethyl-18a-homo-19-nor-androst-4-en-3-on,
17β-Cyano-6β,7β; 15β,16β-bismethylen-18a-homo-19-nor-androst-4-en-3-on,
17β-Cyano-6α,7α; 15β,16β-bismethylen-18a-homo-19-nor-androst-4-en-3-on,
17-Cyano-7α-cyclopropyl-18a-homo-19-nor-androst-4-en-3-on,
17β-Cyano-7β-cyclopropyl-18a-homo-19-nor-androst-4-en-3-on,
17β-Cyano-18a-homo-19-nor-androst-4,6-dien-3-on,
17β₋Cyano-15β,16β-methylen-18a-homo-19-nor-androst-4,6-dien-3-on,
17β-Cyano-7α-vinyl-18a-homo-19-nor-androst-4-en-3-on,
17β-Cyano-7β-vinyl-18a-homo-19-nor-androst-4-en-3-on,
17β-Cyano-17a-methyl-15β,16β-methylen-18a-homo-19-nor-androsta-4,6-dien-3-on,
17β-Cyano-7α,17α-bismethyl-15β,16β-methylen-18a-homo-19-nor-androst-4-en-3-on,
17β-Cyano-7α-ethyl-17α-methyl-15β, 16β-methylen-18a-homo-19-nor-androst-4-en-3-on,
17β-Cyano-7β-ethyl-17α-methyl-15β,16β-methylen-18a-homo-19-nor-androst-4-en-3-on,
17β-Cyano-17α-methyl-15β,16β-methylen-7α-vinyl-18a-homo-19-nor-androst-4-en-3-on,
17β-Cyano-17a-methyl-15β,16β-methylen-7β-vinyl-18a-homo-19-nor-androst-4-en-3-on,
17β-Cyano-7α-cyclopropyl-17α-methyl-15β,16β-methylen-18a-homo-19-norandrost-4-en-3-on,
17β-Cyano-7β-cyclopropyl-17α-methyl-15β,16β-methylen-18a-homo-19-norandrost-4-en-3-on,
17β-Cyano-17α-methyl-6β,7β-15β, 16β-bismethylen-18a-homo-19-norandrost-4-en-3-on,
17β-Cyano-17α-methyl-6a,7a-15β,16β-bismethylen-18a-homo-19-norandrost-4-en-3-on,
17β-Cyano-17α-ethyl-15β, 16β-methylen-18a-homo-19-nor-androsta-4,6-dien-3-on,
17β-Cyano-17α-ethyl-7α-methyl-15β, 16β-methylen-18a-homo-19-nor-androst-4-en-3-on,
17β-Cyano-17α-ethyl-7β-methyl-15β, 16β-methylen-18a-homo-19-nor-androst-4-en-3-on,
17β-Cyano-7α-,17α-bisethyl-15β,16β-methylen-18a-homo-19-nor-androst-4-en-3-on,
17β-Cyano-7β,17α-bisethyl-15β,16β-methylen-18a-homo-19-nor-androst-4-en-3-on,
17β-Cyano-17α-ethyl-15β,16β-methylen-7α-vinyl-18a-homo-19-nor-androst-4-en-3-on,
17β-Cyano-17α-ethyl-15β, 16β-methylen-7β-vinyl-18a-homo-19-nor-androst-4-en-3-on,
17β-Cyano-7α-cyclopropyl-17α-ethyl-15β, 16H-methylen-18a-homo-19-norandrost-4-en-3-on,
17β-Cyano-7β-cyclopropyl-17α-ethyl-15β,16β-methylen-18a-homo-19-norandrost-4-en-3-on,
17β-Cyano-17α-ethyl-6β,7β-15β,16β-bismethylen-18a-homo-19-nor-androst-4-en-3-on und
17β-Cyano-17α-ethyl-6α,7α-15β,16β-bismethylen-18a-homo-19-nor-androst-4-en-3-on.

11. Verwendung des 17β-Cyano-18a-homo-19-nor-androst-4-en-Derivats nach einem der Ansprüche 1 bis 10 zur Herstellung eines Arzneimittels zur oralen Kontrazeption und zur Behandlung von prä-, peri- und postmenopausalen Beschwerden.

12. Verwendung nach Anspruch 11, **dadurch gekennzeichnet, dass** das Arzneimittel gestagene und antimineralcorticoide Wirkung aufweist.

13. Arzneimittel enthaltend mindestens ein 17β-Cyano-18a-homo-19-nor-androst-4-en-Derivat-nach-einem der Ansprüche 1 bis 10 sowie mindestens einen geeigneten pharmazeutisch unbedenklichen Zusatzstoff.

14. Arzneimittel nach Anspruch 13, enthaltend außerdem mindestens ein Estrogen.

15. Arzneimittel nach Anspruch 14, **dadurch gekennzeichnet, dass** das Estrogen Ethinylestradiol ist.

16. Arzneimittel nach Anspruch 14, **dadurch gekennzeichnet, dass** das Estrogen ein natürliches Estrogen ist.

17. Arzneimittel nach Anspruch 16, **dadurch gekennzeichnet, dass** das natürliche Estrogen Estradiol ist.

18. Arzneimittel nach Anspruch 16, **dadurch gekennzeichnet, dass** das natürliche Estrogen Estradiolvalerat ist.

19. Arzneimittel nach Anspruch 16, **dadurch gekennzeichnet, dass** das natürliche Estrogen ein konjugiertes Estrogen ist.

## Claims

1. 17β-Cyano-18a-homo-19-nor-androst-4-ene derivative having the general chemical formula 1 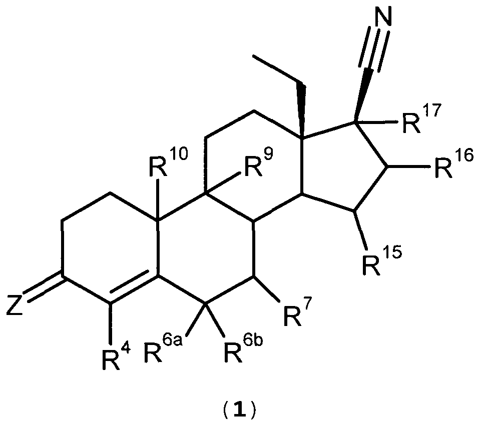 where
Z is selected from the group comprising O, two hydrogen atoms, NOR and NNHSO₂R, in which R is hydrogen or C₁-C₄-alkyl,
R⁴ is hydrogen or halogen,
furthermore either:
R^{6a}, R^{6b} together form methylene or 1,2-ethanediyl or R^{6a} is hydrogen and R^{6b} is selected from the group comprising hydrogen, methyl and hydroxymethylene, and
R⁷ is selected from the group comprising hydrogen, C₁-C₄-alkyl, C₂-C₃-alkenyl and cyclopropyl,
or:
R^{6a} is hydrogen and R^{6b} and R⁷ either together form methylene or are omitted with formation of a double bond between C⁶ and C⁷,
R⁹, R¹⁰ are hydrogen or are omitted with formation of a double bond between C⁹ and C¹⁰,
R¹⁵, R¹⁶ are hydrogen or together form methylene,
R¹⁷ is selected from the group comprising hydrogen, C₁-C₄-alkyl and allyl,
at least one of the substituents R⁴, R^{6a}, R^{6b}, R⁷, R¹⁵, R¹⁶ and R¹⁷ not being hydrogen or R^{6b} and R⁷ being omitted with formation of a double bond between C⁶ and C⁷,
and its solvates, hydrates and salts, where the radicals R^{6a}, R^{6b}, R⁷, R¹⁵ and R¹⁶ and, if present, the methylene group formed by R^{6b} and R⁷ and, if present, the methylene group formed by R¹⁵ and R¹⁶ can be located either in the α- or in the β-position and C₁₋₄-alkyl groups can be perfluorinated.

2. 17β-Cyano-18a-homo-19-nor-androst-4-ene derivative according to Claim 1, **characterized in that** R¹⁵ and R¹⁶ together form methylene.

3. 17β-Cyano-18a-homo-19-nor-androst-4-ene derivative according to one of the above claims, **characterized in that** Z is selected from the group comprising O, NOH and NNHSO₂H.

4. 17β-Cyano-18a-homo-19-nor-androst-4-ene derivative according to one of the above claims, **characterized in that** Z represents O.

5. 17β-Cyano-18a-homo-19-nor-androst-4-ene derivative according to one of the above claims, **characterized in that** R⁴ is hydrogen or chlorine.

6. 17β-Cyano-18a-homo-19-nor-androst-4-ene derivative according to one of the above claims, **characterized in that** R^{6a}, R^{6b} together form 1,2-ethanediyl or are in each case hydrogen.

7. 17β-Cyano-18a-homo-19-nor-androst-4-ene derivative according to one of the above claims, **characterized in that** R⁷ is selected from the group comprising hydrogen and methyl.

8. 17β-Cyano-18a-homo-19-nor-androst-4-ene derivative according to one of Claims 1 to 5, **characterized in that** R^{6b} and R⁷ together form methylene.

9. 17β-Cyano-18a-homo-19-nor-androst-4-ene derivative according to one of the above claims, **characterized in that** R¹⁷ is selected from the group comprising hydrogen and methyl.

10. 17β-Cyano-18a-homo-19-nor-androst-4-ene derivative according to Claim 1, selected from the group comprising
17β-cyano-17α-methyl-15β,16β-methylene-18a-homo-19-nor-androst-4-en-3-one,
17β-cyano-15β,16β-methylene-18a-homo-19-norandrost-4-en-3-one,
17β-cyano-6β-hydroxymethylene-18a-homo-19-norandrost-4-en-3-one,
17β-cyano-6,6-ethanediyl-18a-homo-19-nor-androst-4-en-3-one,
17β-cyano-6β,7β-methylene-18a-homo-19-nor-androst-4-en-3-one,
17β-cyano-6α,7α-methylene-18a-homo-19-nor-androst-4-en-3-one,
17β-cyano-17α-methyl-18a-homo-19-nor-androst-4-en-3-one,
17β-cyano-17α-ethyl-18a-homo-19-nor-androst-4-en-3-one,
17β-cyano-7α-methyl-18a-homo-19-nor-androst-4-en-3-one,
17β-cyano-7α-ethyl-18a-homo-19-nor-androst-4-en-3-one,
17β-cyano-7α-ethyl-18a-homo-19-nor-androst-4-en-3-one,
17β-cyano-6α,7α; 15β,16β-bismethylene-18a-homo-19-nor-androst-4-en-3-one,
17β-cyano-6α,7α; 15β,16β-bismethylene-18a-homo-19-nor-androst-4-en-3-one,
17β-cyano-7α-cyclopropyl-18a-homo-19-nor-androst-4-en-3-one,
17β-cyano-7β-cyclopropyl-18a-homo-19-nor-androst-4-en-3-one,
17β-cyano-18a-homo-19-nor-androst-4,6-dien-3-one,
17β-cyano-15β,16β-methylene-18a-homo-19-norandrosta-4,6-dien-3-one,
17β-cyano-7α-vinyl-18a-homo-19-nor-androst-4-en-3-one,
17β-cyano-7β-vinyl-18a-homo-19-nor-androst-4-en-3-one,
17β-cyano-17α-methyl-15β,16β-methylene-18a-homo-19-nor-androsta-4,6-dien-3-one,
17β-cyano-7α,□17α-bismethyl-15β,16β-methylene-18a-homo-19-nor-androst-4-en-3-one,
17β-cyano-7α-ethyl-17α-methyl-15β,16β-methylene-18a-homo-19-nor-androst-4-en-3-one,
17β-cyano-7β-ethyl-17α-methyl-15β,16β-methylene-18a-homo-19-nor-androst-4-en-3-one,
17β-cyano-17α-methyl-15β,16β-methylene-7α-vinyl-18a-homo-19-nor-androst-4-en-3-one,
17β-cyano-17α-methyl-15β,16β-methylene-7β-vinyl-18a-homo-19-nor-androst-4-en-3-one,
17β-cyano-7α-cyclopropyl-17α-methyl-15β,16β-methylene-18a-homo-19-nor-androst-4-en-3-one,
17β-cyano-7β-cyclopropyl-17α-methyl-15β,16β-methylene-18a-homo-19-nor-androst-4-en-3-one,
17β-cyano-17α-methyl-6β,7β-15β,16β-bismethylene-18a-homo-19-nor-androst-4-en-3-one,
17β-cyano-17α-methyl-6α,7α-15β,16β-bismethylene-18a-homo-19-nor-androst-4-en-3-one,
17β-cyano-17α-ethyl-15β,16β-methylene-18a-homo-19-nor-androsta-4,6-dien-3-one,
17β-cyano-17α-ethyl-7α-methyl-15β,16β-methylene-18a-homo-19-nor-androst-4-en-3-one,
17β-cyano-17α-ethyl-7β-methyl-15β,16β-methylene-18a-homo-19-nor-androst-4-en-3-one,
17β-cyano-7α-,17α-bisethyl-15β,16β-methylene-18a-homo-19-nor-androst-4-en-3-one,
17β-cyano-7β,17α-bisethyl-15β,16β-methylene-18a-homo-19-nor-androst-4-en-3-one,
17β-cyano-17α-ethyl-15β,16β-methylene-7α-vinyl-18a-homo-19-nor-androst-4-en-3-one,
17β-cyano-17α-ethyl-15β,16β-methylene-7β-vinyl-18a-homo-19-nor-androst-4-en-3-one,
17β-cyano-7α-cyclopropyl-17α-ethyl-15β,16β-methylene-18a-homo-19-nor-androst-4-en-3-one,
17β-cyano-7β-cyclopropyl-17α-ethyl-15β,16β-methylene-18a-homo-19-nor-androst-4-en-3-one,
17β-cyano-17α-ethyl-6β,7β-15β,16β-bismethylene-18a-homo-19-nor-androst-4-en-3-one and
17β-cyano-17α-ethyl-6α,7α-15β,16β-bismethylene-18a-homo-19-nor-androst-4-en-3-one.

11. Use of the 17β-cyano-18a-homo-19-nor-androst-4-ene derivatives according to one of Claims 1 to 10 for the production of a medicament for oral contraception and for the treatment of pre-, peri-and postmenopausal symptoms.

12. Use according to Claim 11, **characterized in that** the medicament has gestagenic and antimineralcorticoid action.

13. Medicament comprising at least one 17β-Cyano-18a-homo-19-nor-androst-4-ene derivative according to one of Claims 1 to 10 and at least one suitable pharmaceutically harmless additive.

14. Medicament according to Claim 13, moreover comprising at least one oestrogen.

15. Medicament according to Claim 14, **characterized in that** the oestrogen is ethinylestradiol.

16. Medicament according to Claim 14, **characterized in that** the oestrogen is a natural oestrogen.

17. Medicament according to Claim 16, **characterized in that** the natural oestrogen is oestradiol.

18. Medicament according to Claim 16, **characterized in that** the natural oestrogen is oestradiol valerate.

19. Medicament according to Claim 16, **characterized in that** the natural oestrogen is a conjugated oestrogen.

## Revendications

1. Dérivé de 17β-cyano-18a-homo-19-nor-androst-4-ène de formule chimique générale 1 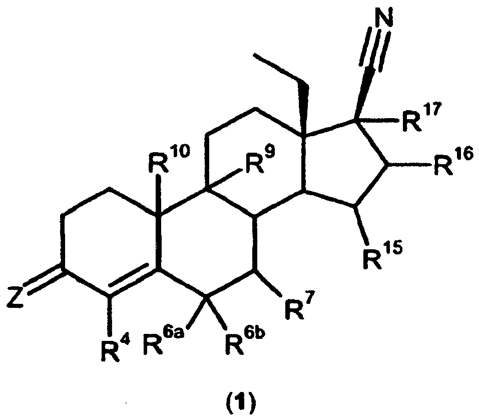 dans laquelle
Z est choisi dans le groupe comprenant O, deux atomes d'hydrogène, NOR et NNHSO₂R, R étant l'hydrogène ou un alkyle en C₁-C₄,
R⁴ représente l'hydrogène ou un halogène,
et soit :
R^{6a}, R^{6b} forment ensemble un méthylène ou un 1,2-éthanediyle, ou R^{6a} représente l'hydrogène et R^{6b} est choisi dans le groupe comprenant hydrogène, méthyle et hydroxyméthylène, et
R⁷ est choisi dans le groupe comprenant hydrogène, alkyle en C₁-C₄, alcényle en C₂-C₃ et cyclopropyle,
soit :
R^{6a} représente l'hydrogène et R^{6b} et R⁷ soit forment ensemble un méthylène, soit sont omis avec formation d'une double liaison entre C⁶ et C⁷,
R⁹, R¹⁰ représentent l'hydrogène ou sont omis avec formation d'une double liaison entre C⁹ et C¹⁰, R¹⁵, R¹⁶ représentent l'hydrogène ou forment ensemble un méthylène,
R¹⁷ est choisi dans le groupe comprenant hydrogène, alkyle en C₁-C₄ et allyle,
au moins un des substituants R⁴, R^{6a}, R^{6b}, R⁷, R¹⁵, R¹⁶ et R¹⁷ étant différent de l'hydrogène ou R^{6b} et R⁷ sont omis avec formation d'une double liaison entre C⁶ et C⁷,
ainsi que ses solvates, hydrates et sels, les radicaux R^{6a}, R^{6b}, R⁷, R¹⁵ et R¹⁶, ainsi que, s'il est présent, le groupe méthylène formé par R^{6b} et R⁷ et, s'il est présent, le groupe méthylène formé par R¹⁵ et R¹⁶, pouvant se trouver en position α ou β, et les groupes alkyle en C₁₋₄ pouvant être perfluorés.

2. Dérivé de 17β-cyano-18a-homo-19-nor-androst-4-ène selon la revendication 1, **caractérisé en ce que** R¹⁵ et R¹⁶ forment ensemble un méthylène.

3. Dérivé de 17β-cyano-18a-homo-19-nor-androst-4-ène selon l'une quelconque des revendications précédentes, **caractérisé en ce que** Z est choisi dans le groupe comprenant O, NOH et NNHSO₂H.

4. Dérivé de 17β-cyano-18a-homo-19-nor-androst-4-ène selon l'une quelconque des revendications précédentes, **caractérisé en ce que** Z représente O.

5. Dérivé de 17β-cyano-18a-homo-19-nor-androst-4-ène selon l'une quelconque des revendications précédentes, **caractérisé en ce que** R⁴ représente l'hydrogène ou le chlore.

6. Dérivé de 17β-cyano-18a-homo-19-nor-androst-4-ène selon l'une quelconque des revendications précédentes, **caractérisé en ce que** R^{6a}, R^{6b} forment ensemble un 1,2-éthanediyle ou représentent chacun l'hydrogène.

7. Dérivé de 17β-cyano-18a-homo-19-nor-androst-4-ène selon l'une quelconque des revendications précédentes, **caractérisé en ce que** R⁷ est choisi dans le groupe comprenant hydrogène et méthyle.

8. Dérivé de 17β-cyano-18a-homo-19-nor-androst-4-ène selon l'une quelconque des revendications 1 à 5, **caractérisé en ce que** R^{6b} et R⁷ forment ensemble un méthylène.

9. Dérivé de 17β-cyano-18a-homo-19-nor-androst-4-ène selon l'une quelconque des revendications précédentes, **caractérisé en ce que** R¹⁷ est choisi dans le groupe comprenant hydrogène et méthyle.

10. Dérivé de 17β-cyano-18a-homo-19-nor-androst-4-ène selon la revendication 1, choisi dans le groupe comprenant :
la 17β-cyano-17α-méthyl-15β,16β-méthylène-18a-homo-19-nor-androst-4-én-3-one,
la 17β-cyano-15β,16β-méthylène-18a-homo-19-nor-androst-4-én-3-one,
la 17β-cyano-6β-hydroxyméthylène-18a-homo-19-nor-androst-4-én-3-one,
la 17β-cyano-6,6-éthanediyl-18a-homo-19-nor-androst-4-én-3-one,
la 17β-cyano-6β,7β-méthylène-18a-homo-19-nor-androst-4-én-3-one,
la 17β-cyano-6α,7α-méthylène-18a-homo-19-nor-androst-4-én-3-one,
la 17β-cyano-17α-méthyl-18a-homo-19-nor-androst-4-én-3-one,
la 17β-cyano-17α-éthyl-18a-homo-19-nor-androst-4-én-3-one,
la 17β-cyano-7α-méthyl-18a-homo-19-nor-androst-4-én-3-one,
la 17β-cyano-7β-éthyl-18a-homo-19-nor-androst-4-én-3-one,
la 17β-cyano-7α-éthyl-18a-homo-19-nor-androst-4-én-3-one,
la 17β-cyano-6β,7β;15β,16β-bisméthylène-18a-homo-19-nor-androst-4-én-3-one,
la 17β-cyano-6α,7α;15β,16β-bisméthylène-18a-homo-19-nor-androst-4-én-3-one,
la 17β-cyano-7α-cyclopropyl-18a-homo-19-nor-androst-4-én-3-one,
la 17β-cyano-7β-cyclopropyl-18a-homo-19-nor-androst-4-én-3-one,
la 17β-cyano-18a-homo-19-nor-androst-4,6-dién-3-one,
la 17β-cyano-15β,16β-méthylène-18a-homo-19-nor-androst-4,6-dién-3-one,
la 17β-cyano-7α-vinyl-18a-homo-19-nor-androst-4-én-3-one,
la 17β-cyano-7β-vinyl-18a-homo-19-nor-androst-4-én-3-one,
la 17β-cyano-17α-méthyl-15β,16β-méthylène-18a-homo-19-nor-androsta-4,6-dién-3-one,
la 17β-cyano-7α,17α-bisméthyl-15β,16β-méthylène-18a-homo-19-nor-androst-4-én-3-one,
la 17β-cyano-7α-éthyl-17α-méthyl-15β,16β-méthylène-18a-homo-19-nor-androst-4-én-3-one,
la 17β-cyano-7β-éthyl-17α-méthyl-15β,16β-méthylène-18a-homo-19-nor-androst-4-én-3-one,
la 17β-cyano-17α-méthyl-15β,16β-méthylène-7α-vinyl-18a-homo-19-nor-androst-4-én-3-one,
la 17β-cyano-17α-méthyl-15β,16β-méthylène-7β-vinyl-18a-homo-19-nor-androst-4-én-3-one,
la 17β-cyano-7α-cyclopropyl-17α-méthyl-15β,16β-méthylène-18a-homo-19-nor-androst-4-én-3-one,
la 17β-cyano-7β-cyclopropyl-17α-méthyl-15β,16β-méthylène-18a-homo-19-nor-androst-4-én-3-one,
la 17β-cyano-17α-méthyl-6β,7β-15β,16β-bisméthylène-18a-homo-19-nor-androst-4-én-3-one,
la 17β-cyano-17α-méthyl-6α,7α-15β,16β-bisméthylène-18a-homo-19-nor-androst-4-én-3-one,
la 17β-cyano-17α-éthyl-15β,16β-méthylène-18a-homo-19-nor-androsta-4,6-dién-3-one,
la 17β-cyano-17α-éthyl-7α-méthyl-15β,16β-méthylène-18a-homo-19-nor-androst-4-én-3-one,
la 17β-cyano-17α-éthyl-7β-méthyl-15β,16β-méthylène-18a-homo-19-nor-androst-4-én-3-one,
la 17β-cyano-7α,17α-biséthyl-15β,16β-méthylène-18a-homo-19-nor-androst-4-én-3-one,
la 17β-cyano-7β,17α-biséthyl-15β,16β-méthylène-18a-homo-19-nor-androst-4-én-3-one,
la 17β-cyano-17α-éthyl-15β,16β-méthylène-7α-vinyl-18a-homo-19-nor-androst-4-én-3-one,
la 17β-cyano-17α-éthyl-15β,16β-méthylène-7β-vinyl-18a-homo-19-nor-androst-4-én-3-one,
la 17β-cyano-7α-cyclopropyl-17α-éthyl-15β,16β-méthylène-18a-homo-19-nor-androst-4-én-3-one,
la 17β-cyano-7β-cyclopropyl-17α-éthyl-15β,16β-méthylène-18a-homo-19-nor-androst-4-én-3-one,
la 17β-cyano-17α-éthyl-6β,7β-15β,16β-bisméthylène-18a-homo-19-nor-androst-4-én-3-one et
la 17β-cyano-17α-éthyl-6α,7α-15β,16β-bisméthylène-18a-homo-19-nor-androst-4-én-3-one.

11. Utilisation du dérivé de 17β-cyano-18a-homo-19-nor-androst-4-ène selon l'une quelconque des revendications 1 à 10 pour la fabrication d'un médicament pour la contraception orale ou pour le traitement des troubles de la pré-, péri- et post-ménopause.

12. Utilisation selon la revendication 11, **caractérisée en ce que** le médicament présente un effet gestagène et antiminéralocorticoïde.

13. Médicament contenant au moins un dérivé de 17β-cyano-18a-homo-19-nor-androst-4-ène selon l'une quelconque des revendications 1 à 10, ainsi qu'au moins un additif pharmaceutiquement inoffensif approprié.

14. Médicament selon la revendication 13, contenant également au moins un estrogène.

15. Médicament selon la revendication 14, **caractérisé en ce que** l'estrogène est l'éthinylestradiol.

16. Médicament selon la revendication 14, **caractérisé en ce que** l'estrogène est un estrogène naturel.

17. Médicament selon la revendication 16, **caractérisé en ce que** l'estrogène naturel est l'estradiol.

18. Médicament selon la revendication 16, **caractérisé en ce que** l'estrogène naturel est le valérate d'estradiol.

19. Médicament selon la revendication 16, **caractérisé en ce que** l'estrogène naturel est un estrogène conjugué.
